# EUROPEAN PATENT APPLICATION

(11) **EP 3 939 999 A1**
(43) Date of publication of application: **19.01.2022**
(21) Application number: 20382631.8
(22) Date of filing: 14.07.2020
(51) Int. Cl.: C07K 16/28, A61P 35/00

(54) **INTERLEUKIN 11 RECEPTOR ALPHA SUBUNIT (IL11RA) NEUTRALIZING ANTIBODIES AND USES THEREOF**

(71) Applicant: Fundación del Sector Público Estatal Centro Nacional de Investigaciones Oncológicas Carlos III (F.S.P. CNIO), 28029 Madrid (ES); Fundación Investigación Biomédica Hospital Universitario 12 de Octubre, 28041 Madrid (ES)
(72) Inventor: OJEDA MÁRQUEZ, Laura, 41640 Osuna, Sevilla (ES); FERRER SÁNCHEZ, Irene, 28007 Madrid (ES); PAZ-ARES RODRÍGUEZ, Luis, 28003 Madrid (ES); MARTÍNEZ TORRECUADRADA, Jorge Luis, 28660 Boadilla del Monte, Madrid (ES); RONCADOR, Giovanna, 28029 Madrid (ES)
(74) Representative: ABG Intellectual Property Law, S.L.

(57) **Abstract**

The present invention relates to neutralizing antibodies which specifically recognize the interleukin 11 receptor alpha subunit (IL11RA) extracellular domain and to antibody drug conjugates (ADCs) comprising a therapeutic agent and said neutralizing antibodies. The invention also relates to a method for detecting the IL-11 receptor, to pharmaceutical compositions and to the use of said antibodies or ADCs in the treatment of diseases characterized by an increased IL11RA signaling.

SEQUENCE LISTING

<110> FUNDACIÓN DEL SECTOR PÚBLICO ESTATAL CENTRO NACIONAL DE
      INVESTIGACIONES ONCOLÓGICAS CARLOS III (F.S.P. CNIO)
      FUNDACIÓN INVESTIGACIÓN BIOMÉDICA HOSPITAL UNIVERSITARIO 12
       DE OCTUBRE

<120> INTERLEUKIN 11 RECEPTOR ALPHA SUBUNIT (IL11RA) NEUTRALIZING
      ANTIBODIES AND USES THEREOF

<130> P19550EP00

<160> 16

<170> PatentIn version 3.5

<210> 1
<211> 5
<212> PRT
<213> Artificial Sequence

<220>
<223> VH_CDR1

<400> 1

Asp Tyr Ser Val His
1               5


<210> 2
<211> 14
<212> PRT
<213> Artificial Sequence

<220>
<223> VH_CDR2

<400> 2

Arg Met Lys Phe Asp Gly Asp Thr His Tyr Asn Ser Ala Leu
1               5                   10


<210> 3
<211> 11
<212> PRT
<213> Artificial Sequence

<220>
<223> VH_CDR3

<400> 3

Glu Gly Tyr Tyr Asp Gly Thr Trp Gly Trp Tyr
1               5                   10


<210> 4
<211> 11
<212> PRT
<213> Artificial Sequence

<220>
<223>  VL_CDR1

<400>  4

Lys Ala Ser Gln Asn Ile Asn Lys Tyr Leu Asn
1               5                   10


<210>  5
<211>  7
<212>  PRT
<213>  Artificial Sequence

<220>
<223>  VL_CDR2

<400>  5

Asn Thr Asn Ser Leu Gln Thr
1               5


<210>  6
<211>  9
<212>  PRT
<213>  Artificial Sequence

<220>
<223>  VL_CDR3

<400>  6

Phe Gln His Asn Ser Trp Pro Leu Thr
1               5


<210>  7
<211>  30
<212>  PRT
<213>  Artificial Sequence

<220>
<223>  VH_FR1

<400>  7

Gln Val Gln Leu Lys Glu Ser Gly Pro Gly Leu Val Gln Pro Ser Lys
1               5                   10                  15


Thr Leu Ser Leu Thr Cys Thr Val Ser Gly Phe Ser Leu Met
            20                  25                  30


<210>  8
<211>  14
<212>  PRT
<213>  Artificial Sequence

<220>
<223>  VH_FR2

<400>  8

Trp Val Arg Gln Pro Pro Gly Gln Gly Leu Glu Trp Met Gly
1               5                   10

<210>  9
<211>  34
<212>  PRT
<213>  Artificial Sequence

<220>
<223>  VH_FR3

<400>  9

Lys Ser Arg Leu Ser Ile Arg Arg Asp Thr Ser Lys Ser Gln Val Phe
1               5                   10                  15

Leu Arg Met Asn Ser Val Gln Thr Glu Asp Thr Ala Ile Tyr Tyr Cys
            20                  25                  30

Asn Arg

<210>  10
<211>  11
<212>  PRT
<213>  Artificial Sequence

<220>
<223>  VH_FR4

<400>  10

Trp Gly Gln Gly Val Lys Val Thr Val Ser Ser
1               5                   10

<210>  11
<211>  23
<212>  PRT
<213>  Artificial Sequence

<220>
<223>  VL_FR1

<400>  11

Asp Val Gln Met Thr Gln Ser Pro Ser Leu Leu Ser Ala Ser Val Gly
1               5                   10                  15

Asp Arg Val Thr Ile Asn Cys
            20

<210>  12
<211>  15
<212>  PRT

<213>  Artificial Sequence

<220>
<223>  VL_FR2

<400>  12

Trp Tyr Gln Gln Lys Leu Gly Glu Ala Pro Lys Leu Leu Ile Tyr
1               5                   10                  15


<210>  13
<211>  32
<212>  PRT
<213>  Artificial Sequence

<220>
<223>  VL_FR3

<400>  13

Gly Ile Pro Ser Arg Phe Ser Gly Ser Gly Ser Gly Thr Asp Tyr Thr
1               5                   10                  15


Leu Thr Ile Phe Ser Leu His Pro Glu Asp Phe Gly Thr Tyr Phe Cys
            20                  25                  30


<210>  14
<211>  10
<212>  PRT
<213>  Artificial Sequence

<220>
<223>  VL_FR4

<400>  14

Phe Gly Ser Gly Thr Lys Leu Glu Ile Lys
1               5                   10


<210>  15
<211>  119
<212>  PRT
<213>  Artificial Sequence

<220>
<223>  VH

<400>  15

Gln Val Gln Leu Lys Glu Ser Gly Pro Gly Leu Val Gln Pro Ser Lys
1               5                   10                  15


Thr Leu Ser Leu Thr Cys Thr Val Ser Gly Phe Ser Leu Met Asp Tyr
            20                  25                  30


Ser Val His Trp Val Arg Gln Pro Pro Gly Gln Gly Leu Glu Trp Met
        35                  40                  45

Gly Arg Met Lys Phe Asp Gly Asp Thr His Tyr Asn Ser Ala Leu Lys
    50                  55                  60

Ser Arg Leu Ser Ile Arg Arg Asp Thr Ser Lys Ser Gln Val Phe Leu
65                  70                  75                  80

Arg Met Asn Ser Val Gln Thr Glu Asp Thr Ala Ile Tyr Tyr Cys Asn
                85                  90                  95

Arg Glu Gly Tyr Tyr Asp Gly Thr Trp Gly Trp Tyr Trp Gly Gln Gly
            100                 105                 110

Val Lys Val Thr Val Ser Ser
        115

<210>  16
<211>  107
<212>  PRT
<213>  Artificial Sequence

<220>
<223>  VL

<400>  16

Asp Val Gln Met Thr Gln Ser Pro Ser Leu Leu Ser Ala Ser Val Gly
1               5                   10                  15

Asp Arg Val Thr Ile Asn Cys Lys Ala Ser Gln Asn Ile Asn Lys Tyr
            20                  25                  30

Leu Asn Trp Tyr Gln Gln Lys Leu Gly Glu Ala Pro Lys Leu Leu Ile
        35                  40                  45

Tyr Asn Thr Asn Ser Leu Gln Thr Gly Ile Pro Ser Arg Phe Ser Gly
    50                  55                  60

Ser Gly Ser Gly Thr Asp Tyr Thr Leu Thr Ile Phe Ser Leu His Pro
65                  70                  75                  80

Glu Asp Phe Gly Thr Tyr Phe Cys Phe Gln His Asn Ser Trp Pro Leu
                85                  90                  95

Thr Phe Gly Ser Gly Thr Lys Leu Glu Ile Lys
            100                 105

## Description

### FIELD OF THE INVENTION

The present invention is comprised within the field of biotechnology and biomedicine. It specifically relates to antibodies specific against interleukin 11 receptor alpha subunit (IL11RA) as well as to antibody-drug conjugates and the uses thereof in in the treatment of cancer and other pathologies which require inhibiting IL-11 mediated signaling such as fibroinflammatory diseases.

### BACKGROUND OF THE INVENTION

Cancer is one of the leading causes of morbidity and mortality worldwide. It is now responsible for almost one in six deaths globally and the number of new cases is expected to rise by about 70% over the next 2 decades.

Many drugs are now available to be used in the treatment of cancer. However, in many cases the cancer fails to respond to the anti-cancer therapy or its growth and/or metastasis is only slowed. Even when a tumor initially responds to an anti-cancer therapy by decreasing in size or going into remission, the tumor often develops resistance to the drug. For these reasons, there is a need for new anti-cancer agents and drugs which can be used to treat cancers for which there is still no treatment available and for multi-drug resistance cancers.

Among cancers, lung cancer is the first cause of cancer related deaths worldwide due to its high incidence, frequent late diagnosis and the limited efficacy of available therapies. In last two decades the development of personalized therapies for lung cancer had had an important impact on the survival of these patients. However, there is an urgent unmet need to find tailored treatment alternatives for more patients.

### SUMMARY OF THE INVENTION

In a first aspect the invention relates to a neutralizing antibody which specifically recognizes the interleukin 11 receptor alpha subunit (IL11RA) extracellular domain, wherein said antibody comprises:
- a heavy chain variable region (VH) wherein the CDR1, CDR2 and CDR3 of the VH comprise, respectively, the sequences of SEQ ID NO: 1, 2 and 3 or functionally equivalent variants thereof, and
- a light chain variable region (VL) wherein the CDR1, CDR2 and CDR3 of the VL region comprise respectively, the sequences of SEQ ID NO: 4, 5, and 6 or functionally equivalent variants thereof.

In a second aspect, the invention relates to a nucleic acid encoding an antibody according to the first aspect of the invention.

In a third aspect, the invention relates to an expression vector comprising the nucleic acid of the second aspect of the invention.

In a fourth aspect, the invention relates to a host cell comprising the nucleic acid of the second aspect of the invention or the expression vector of the third aspect of the invention.

In a fifth aspect, the invention relates to an antibody-drug conjugate (ADC) comprising:
(i) a therapeutic agent, and
(ii) an antibody according to any of claims 1 to 5.

In a sixth aspect, the invention relates to a method for detecting the IL-11 receptor in a sample which comprises contacting the sample with an antibody according to claim 1 coupled to a detectable label and detecting in said sample the formation of a complex between the antibody and the IL-11 receptor.

In a seventh aspect, the invention relates to a pharmaceutical composition comprising the antibody according the first aspect of the invention or the ADC of the fifth aspect of the invention and at least one pharmaceutically acceptable excipient and/or vehicle.

In an eight aspect, the invention relates to the antibody of the first aspect of the invention or the ADC of the fifth aspect of the invention for use in medicine.

In an ninth aspect, the invention relates to the antibody or the ADC of the invention for use in a method of preventing or treating a disease characterized by increased IL11 signaling, wherein the disease is selected from pulmonary fibrosis, liver fibrosis, liver inflammation, steatosis, steatohepatitis, NASH and cancer. In a final aspect, the invention relates to a neutralizing antibody which specifically recognizes the interleukin 11 receptor alpha subunit (IL11RA) extracellular domain for use in a method of preventing or treating any of the aforementioned diseases and, more in particular, lung cancer.

### DESCRIPTION OF THE FIGURES

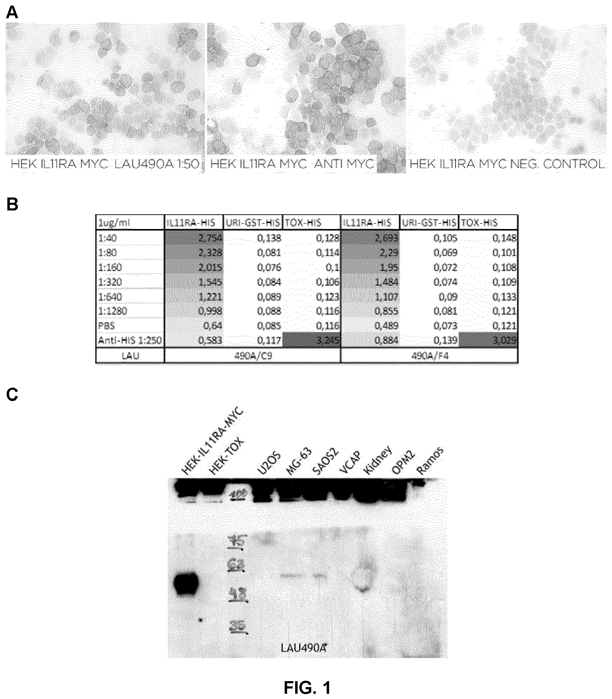

**Figure 1****.** *Characterization of the LAU490A antibody.* (A). Immunocytochemistry on HEK-IL11RA-MYC transfected cells with LAU490A antibody. (B). ELISA of LAU490A antibodies with recombinant IL11RA protein, URI-GST-HIS and TOX-HIS were used as a negative controls. (C). Western blotting of LAU490A antibody in different human protein extracts and human cell lines.

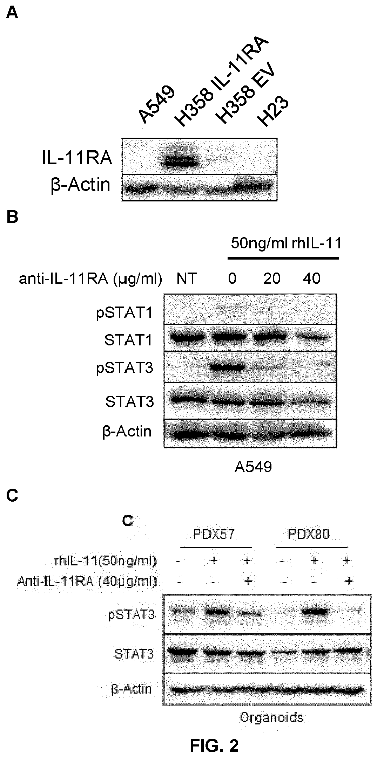

**Figure 2****.** *In vitro characterization of IL-11RA neutralizing antibody.* IL-11RA antibody was validated for its efficacy detecting IL-11RA by WB, using lung adenocarcinoma cells such as A549 and H23, as well as the cell line H358 with overexpression of IL-11RA or expressing the empty vector as control (A). In order to analyze its neutralizing effect, A549 cells (B) and organoids from PDXs models 57 and 80 (C) were pre-treated with the IL-11RA antibody and then stimulated with 50ng/ml of rhIL-11. By WB, the phosphorylation of proteins STAT1 and STAT3 was studied. As a result, after the stimulation of cells and organoids with rhIL-11 there was increased the phosphorylation of STAT proteins and this phosphorylation was inhibited when treating with IL-11RA. WB=Western Blot, H358 IL-11RA=H358 cell line with overexpression of IL-11RA, H358 EV=H358 cell line with expression of the EV pCMV6, NT=non-treated, PDX=Patient Derived Xenograft, rhIL-11=Recombinant human IL-11.

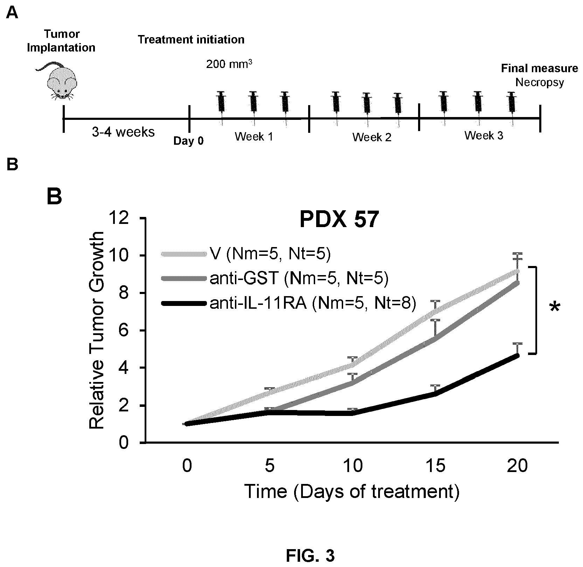

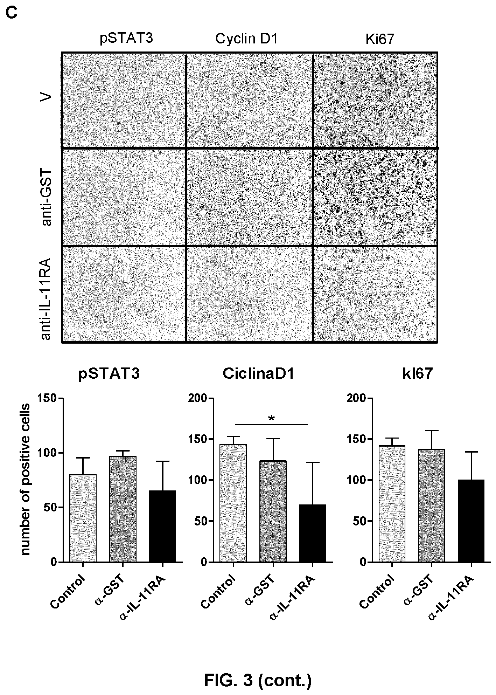

**Figure 3****.** *In vivo characterization of IL-11RA neutralizing antibody.* Tumors of PDX model were implanted in flanks of athymic mice and when they reached a size of 200 mm3 they were treated with anti-IL-11RA (300 µg/mice), anti-GST (300 µg/mice) or vehicle by intraperitoneal injection, 3 times a week for 3 weeks (A). Tumor growth was measured by caliper weekly and the relative growth was calculated by normalizing the tumor size at the end of the procedure with the size in day 0 of treatment (B). The graphs show the expression of the pSTAT3, Ki67 and Cyclin D1 of each group (n=3) analyzed by IHQ staining after treatment. Representative image showing the staining of each protein are displayed on the right side (C).The statistical analysis performed was Kruskal-Wallis test (*p <0.05, **p <0.01, ***p <0.001). V=vehicle, Nm=Number of mice in each group, Nt=Number of tumors implanted in each group, PDX=Patient Derived Xenograft.

### DETAILED DESCRIPTION OF THE INVENTION

The present invention relates to the provision of new compounds for the treatment of - diseases associated with an excessive IL-11-mediated diseases and, in particular, cancer.

Unless otherwise defined, all technical terms used herein have the same meaning as commonly understood by one of ordinary skill in the art to which this invention belongs. All the embodiments and definitions disclosed in the context of one aspect of the invention are also applicable to the other aspects of the invention.

### Antibodies

The authors of the present invention have obtained an antibody which specifically binds to interleukin 11 receptor alpha subunit (IL11RA) extracellular domain and which is capable to block the IL-11 mediated signaling pathway *in vitro* (see Example 2) and to elicit antitumor activity *in vivo* (see Example 3 and figure 3).

Thus, in a first aspect, the invention relates to a neutralizing antibody which specifically recognizes the interleukin 11 receptor alpha subunit (IL11RA) extracellular domain, wherein said antibody comprises:
- a heavy chain variable region (VH) wherein the CDR1, CDR2 and CDR3 of the VH comprise, respectively, the sequences of SEQ ID NO: 1, 2 and 3 or functionally equivalent variants thereof, and
- a light chain variable region (VL) wherein the CDR1, CDR2 and CDR3 of the VL region comprise respectively, the sequences of SEQ ID NO: 4, 5, and 6 or functionally equivalent variants thereof.

The term "antibody", as used herein, refers to an immunoglobulin molecule or a fragment of an immunoglobulin molecule which has the ability to specifically bind to an epitope of a molecule ("antigen"). Naturally occurring antibodies typically comprise a tetramer which is usually composed of at least two heavy (H) chains and at least two light (L) chains. Each heavy chain is comprised of a heavy chain variable domain (abbreviated herein as VH) and a heavy chain constant domain, usually comprised of three domains (CH1, CH2 and CH3). Heavy chains are classified as γ, µ, α, δ and ε, and they define the isotype of the antibody as respectively IgG, IgM, IgA, IgD or IgE. Each light chain is comprised of a light chain variable domain (abbreviated herein as VL) and a light chain constant domain (CL). Light chains include kappa chains and lambda chains (K or λ). The heavy and light chain variable domain is typically responsible for antigen recognition, while the heavy and light chain constant domain may mediate the binding of the immunoglobulin to host tissues or factors, including various cells of the immune system (e.g., effector cells) and the first component (C1 q) of the classical complement system. The VH and VL domains can be further subdivided into domains of hypervariability, termed "complementarity determining regions" (CDR) that are interspersed with domains of more conserved sequence, termed "framework regions" (FR). Each VH and VL is composed of three CDR domains and four FR domains arranged from amino-terminus to carboxy-terminus in the following order: FR1 -CDR1 -FR2- CDR2-FR3-CDR3-FR4. The variable domains of the heavy and light chains contain a binding domain that interacts with an antigen. Of particular relevance are antibodies and their epitope-binding fragments that have been "isolated" so as to exist in a physical milieu distinct from that in which it may occur in nature or that have been modified so as to differ from a naturally occurring antibody in amino acid sequence.

The term "neutralizing", as used herein, refers to an antibody which is capable of neutralizing the biological signaling activity of IL-11 for example by blocking binding of IL-11 to its corresponding receptor. It will be appreciated that the term 'neutralising' as used herein refers to a reduction in biological signaling activity which may be partial or complete. In particular a neutralizing antibody according to the invention refers to an antibody which inhibits (partially or completely) the oncogenic properties of IL-11. For example, said neutralizing activities are typically evaluated according to the assay described in Example 2 and, in particular, by determining the capacity of the antibody to inhibit the activation of the JAK/STAT pathway in cells or organoids expressing the IL-11 receptor after being contacted with IL-11. The neutralizing antibody according to the invention is capable of neutralizing at least 50%, at least 55%, at least 60%, at least 65 %, at least 70%, at least 75%, at least 80%, at least 85%, at least 90%, at least 91 %, at least 92%, at least 93%, at least 94%, at least 95%, at least 96%, at least 97%, at least 98%, at least 99% or at least 100% of the IL-11-mediated activity when determined using any standard assay for determining IL-11-mediated signaling. In a preferred embodiment, the IL-11-medianted signaling is determined as described in Example 2, i.e. by determining the capacity of the antibody to block the activation of the JAK/STAT pathway in cells or organoids expressing the IL-11 receptor after being contacted with IL-11. In yet another embodiment, the activation of the JAK/STAT pathway is determined by measuring the levels of the STAT1 and/or STAT3 phosphorylation in those cells.

The term "variable region" or "variable domain" refers to the domain of an antibody heavy or light chain that is involved in binding the antibody to antigen. The variable domains of the heavy chain and light chain (VH and VL, respectively) of a native antibody generally have similar structures, with each domain comprising four conserved framework regions (FRs) and three hypervariable regions (HVRs) or complementary determining regions (CDRs). A single VH or VL domain may be sufficient to confer antigen-binding specificity.

The term "hypervariable region", "HVR", "complementarity determining regions" or "CDRs" as used herein, refers to each of the regions of an antibody variable domain which are hypervariable in sequence and/or form structurally defined loops ("hypervariable loops"). Generally, native four-chain antibodies comprise six CDRs; three in the VH (H1, H2, H3), and three in the VL (L1, L2, L3). Thus, CDRs determine the protein's affinity (roughly, bonding strength) and specificity for specific antigens. The CDRs of the two chains of each pair are aligned by the framework regions, acquiring the function of binding a specific epitope. Consequently, both the heavy variable chain and the light variable chain are characterized by three CDRs, respectively VH-CDR1, VH-CDR2, VH-CDR3 and VL-CDR1, VL-CDR2, VL-CDR3.

The CDR sequences can be determined according to conventional criteria, for example by means of the criteria of IgBLAST: http://www.ncbi.nlm.nih.gov/igblast/ (Ye et al., 2013, Nucleic Acids Res 41 (Web Server issue:W34-40), by following the numbering provided by Kabat et al, Sequences of Proteins of Immunological Interest, 5th ed.,Public Health Service, National Institutes of Health, Bethesda, Md. (1991), or by following the numbering provided by Chothia et al. (1989, Nature 342:877-83).This particular region has been described by Kabat et al., Sequences of Proteins of Immunological Interest, 5th Ed. Public Health Service, National Institutes of Health, Bethesda, Md. (1991) and by Chothia et al., J. Mol. Biol. 196:901-917 (1987), where the definitions include overlapping or subsets of amino acid residues when compared against each other. The exact residue numbers which encompass a particular CDR will vary depending on the sequence and size of the CDR. Those skilled in the art can routinely determine which residues comprise a particular CDR given the variable region amino acid sequence of the antibody. The CDR sequences given herein are generally according to the Kabat definition.

"Framework" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following order in VH (or VL): FR1-H1 (L1)-FR2-H2(L2)-FR3-H3 (L3)-FR4.

The term antibody comprises whole monoclonal antibodies or polyclonal antibodies, or fragments thereof, that retain one or more CDR regions, and includes human antibodies, humanized antibodies, chimeric antibodies and antibodies of a non-human origin.

"Monoclonal antibodies" are homogenous, highly specific antibody populations directed against a single site or antigenic "determinant". "Polyclonal antibodies" include heterogeneous antibody populations directed against different antigenic determinants.

In a particular embodiment, the antibody of the invention is an antibody of non-human origin, preferably of murine origin. In a preferred embodiment, the antibody of the invention is a monoclonal antibody.

As used herein, the antibody of the invention encompasses not only full length antibodies (e.g., IgG), but also antigen-binding fragments thereof, for example, Fab, Fab', F(ab')2, Fv fragments, human antibodies, humanized antibodies, chimeric antibodies, antibodies of a non-human origin, recombinant antibodies, and polypeptides derived from immunoglobulins produced by means of genetic engineering techniques, for example, single chain Fv (scFv), diabodies, heavy chain or fragments thereof, light chain or fragment thereof, VH or dimers thereof, VL or dimers thereof, Fv fragments stabilized by means of disulfide bridges (dsFv), molecules with single chain variable region domains (Abs), minibodies, scFv-Fc, VL and VH domains and fusion proteins comprising an antibody, or any other modified configuration of the immunoglobulin molecule that comprises an antigen recognition site of a desired specificity. The antibody of the invention may also be a bispecific antibody. An antibody fragment may refer to an antigen binding fragment.

As used herein a "recombinant antibody" is an antibody that comprises an amino acid sequence derived from two different species or, from two different sources, and includes synthetic molecules, for example, an antibody that comprises a non-human CDR and a human framework or constant region. In certain embodiments, recombinant antibodies of the present invention are produced from a recombinant DNA molecule or synthesized.

The person skilled in the art will understand that the amino acid sequences of the antibodies of the invention can include one or more amino acid substitutions such that, even though the primary sequence of the polypeptide is altered, the capacity of the antibody to bind to the interleukin 11 receptor alpha subunit (IL11RA) antigen is maintained. Said substitution can be a conservative substitution and is generally applied to indicate that the substitution of one amino acid with another amino acid with similar properties (for example, the substitution of glutamic acid (negatively charged amino acid) with aspartic acid would be a conservative amino acid substitution).

Amino acid sequence modification(s) of the antibody described herein are contemplated. For example, it may be desirable to improve the binding affinity and/or other biological properties of the antibody. Amino acid sequence variants of the antibody are prepared by introducing appropriate nucleotide changes into the antibody encoding nucleic acid, or by peptide synthesis. Such modifications include, for example, deletions from, and/or insertions into and/or substitutions of, residues within the amino acid sequences of the antibody. Any combination of deletion, insertion, and substitution is made to achieve the final construct, provided that the final construct possesses the desired characteristics. The amino acid changes may also alter post-translational processes of the protein, such as changing the number or position of glycosylation sites.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include a peptide with an N-terminal methionyl residue or the antibody polypeptidic chain fused to a cytotoxic polypeptide. Other insertional variants of the molecule include the fusion to the N- or C-terminus of an enzyme, or a polypeptide which increases its serum half-life.

Another type of variant is an amino acid substitution variant. These variants have at least one amino acid residue in the molecule replaced by a different residue. The sites of greatest interest for substitution mutagenesis of antibodies include the hypervariable regions.

The term "specifically binds", "specific binding" or "specifically recognizes", when used in the present invention to refer to the binding of an antibody or a fragment thereof to the interleukin 11 receptor alpha subunit (IL11RA) extracellular domain, is understood as the capacity of the antibody or fragment thereof to bind specifically to the IL11RA extracellular domain by means of the existence of complementarity between the three-dimensional structures of the two molecules with a substantially higher affinity for nonspecific binding such that the binding between said antibody or fragment thereof and the IL11RA extracellular domain preferably takes place before the binding of any of said molecules with respect to the other molecules present in the reaction mixture. This results in that the antibody or fragment thereof does not cross-react with other molecules. Cross-reactivity of the antibody or fragment thereof may be tested, for example, by assessing binding of said antibody or fragment thereof under conventional conditions to the protein of interest as well as to a number of more or less (structurally and/or functionally) closely related proteins. For instance, a binding can be considered specific if the binding affinity between the antibody and the IL11RA extracellular domain has a dissociation constant (KD) of less than 10⁻⁶ M, less than 10⁻⁷ M, less than 10⁻⁸ M, less than 10⁻⁹ M, less than 10⁻¹⁰ M, less than 10⁻¹¹ M, less than 10⁻¹² M, less than 10⁻¹³ M, less than 10⁻¹⁴ M or less than 10⁻¹⁵ M.

The capacity of the antibody or antibody fragment as described herein, to bind to the IL11RA extracellular domain can be determined by a number of assays that are well known in the art. Preferably, the binding capacity of the binding agents is determined by immunoprecipitation or by an in vitro binding assay, such as radioimmunoassay (RIA), enzyme-linked immunoabsorbent assay (ELISA), surface plasmon resonance or by immunofluorescent techniques such as immunohistochemistry (IHC), fluorescence microscopy or flow cytometry.

The term "interleukin 11 receptor alpha subunit (IL11RA)" refers to a human gene which codes for the subunit of the interleukin 11 receptor. Interleukin 11 is a stromal cell-derived cytokine that belongs to a family of pleiotropic and redundant cytokines that use the gp130 transducing subunit in their high affinity receptors. This gene encodes the IL-11 receptor, which is a member of the hematopoietic cytokine receptor family. This particular receptor is very similar to ciliary neurotrophic factor, since both contain an extracellular region with a 2-domain structure composed of an immunoglobulin-like domain and a cytokine receptor-like domain. Alternative splicing has been observed at this locus and two variants, each encoding a distinct isoform, have been identified. The human gene is shown in the Ensembl database under accession number ENSG00000137070 (Ensembl release 100, April 2020).

The term "extracellular domain" refers to the part of the receptor which protrudes from the outer membrane of the cell organelles and cells.

The antibody of the invention comprises; a heavy chain variable region (VH) wherein the CDR1, CDR2 and CDR3 of the VH comprise respectively the sequences of SEQ ID NO: 1, 2 and 3 or functionally equivalent variants; and a light chain variable region (VL) wherein the CDR1, CDR2 and CDR3 of the VL region comprise respectively the sequences of SEQ ID NO: 4, 5 and 6 or functionally equivalent variants thereof.

As it is used herein, the term "functionally equivalent variant of a CDR sequence" refers to a sequence variant of a particular CDR sequence having substantially similar sequence identity with it and substantially maintaining its capacity to bind to its cognate antigen when being part of an antibody, antibody fragment or antigen-binding domain as the ScFv described herein. For example, a functionally equivalent variant of a CDR sequence may be a polypeptide sequence derivative of said sequence comprising the addition, deletion or substitution of one or more amino acids. In one embodiment, the substitution of one amino acid by other in the functionally equivalent variant is a conservative substitution.

As used herein, the term "conservative substitution" refers to the replacement of an amino acid by another amino acid having similar chemical properties. Conservative substitution tables providing functionally similar amino acids are well known in the art. The following six groups each contain amino acids that are conservative substitutions for one another:
- Alanine (A), Serine (S), Threonine (T);
- Aspartic acid (D), Glutamic acid (E);
- Asparagine (N), Glutamine (Q);
- Arginine (R), Lysine (K);
- Isoleucine (I), Leucine (L), Methionine (M), Valine (V); and
- Phenylalanine (F), Tyrosine (Y), Tryptophan (W).

Functionally equivalent variants of a CDR sequence according to the invention include CDR sequences having at least 70% %, at least 75%, at least 80%, at least 85%, at least 90%, at least 91 %, at least 92%, at least 93%, at least 94%, at least 95%, at least 96%, at least 97%, at least 98% or at least 99% sequence identity with the corresponding amino acid sequences shown in one of SEQ ID NOs 1 to 6. It is also contemplated that functionally equivalent variants of a CDR sequence comprise additions consisting of at least 1 amino acid, or at least 2 amino acids, or at least 3 amino acids, or at least 4 amino acids, or at least 5 amino acids, or at least 6 amino acids, or at least 7 amino acids, or at least 8 amino acids, or at least 9 amino acids, or at least 10 amino acids or more amino acids at the N-terminus, or at the C-terminus, or both at the N- and C-terminus of the corresponding amino acid sequence shown in one of above referenced sequences. Likewise, it is also contemplated that variants comprise deletions consisting of at least 1 amino acid, or at least 2 amino acids, or at least 3 amino acids, or at least 4 amino acids, or at least 5 amino acids, or at least 6 amino acids, or at least 7 amino acids, or at least 8 amino acids, or at least 9 amino acids, or at least 10 amino acids or more amino acids at the N-terminus, or at the C-terminus, or both at the N- and C-terminus of the corresponding amino acid sequence shown in one of the above mentioned sequences.

Functionally equivalent variants of a CDR sequence according to the invention will preferably maintain at least 70%, at least 75%, at least 80%, at least 85%, at least 90%, at least 91 %, at least 92%, at least 93%, at least 94%, at least 95%, at least 96%, at least 97%, at least 98%, at least 99%, at least 100%, at least 105%, at least 1 10%, at least 1 15%, at least 120%, at least 125%, at least 130%, at least 135%, at least 140%, at least 145%, at least 150%, at least 200% or more of the capacity of the corresponding amino acid sequence shown in one of SEQ ID NOs: 1 to 6 to bind to its cognate antigen when being part of the antibody or antibody fragment of the invention. This capacity to bind to its cognate antigen may be determined as a value of affinity, avidity, specificity and/or selectivity of the antibody or antibody fragment to its cognate antigen.

In an embodiment, the FR1, FR2, FR3 and FR4 of the VH region of the antibody of the invention comprise respectively the sequences of SEQ ID NO: 7, 8, 9 and 10 or functionally equivalent variants thereof and/or the FR1, FR2, FR3 and FR4 of the VL region of the antibody of the invention comprise respectively the sequences of SEQ ID NO: 11, 12, 13 and 14 or functionally equivalent variants thereof

As it is used herein, the term "functionally equivalent variant of a FR sequence" refers to a sequence variant of a particular FR sequence having substantially similar sequence identity with it and substantially maintaining its capacity to bind to its cognate antigen when being part of an antibody or antibody-binding domains described herein. For example, a functionally equivalent variant of a FR sequence may be a polypeptide sequence derivative of said sequence comprising the addition, deletion or substitution of one or more amino acids.

Functionally equivalent variants of a FR sequence according to the invention include FR sequences having at least approximately 70% , at least 75%, at least 80%, at least 85%, at least 90%, at least 91 %, at least 92%, at least 93%, at least 94%, at least 95%, at least 96%, at least 97%, at least 98% or at least 99% sequence identity with the corresponding amino acid sequences shown in one of SEQ ID NO: 7-13. It is also contemplated that functionally equivalent variants of a FR sequence comprise additions consisting of at least 1 amino acid, or at least 2 amino acids, or at least 3 amino acids, or at least 4 amino acids, or at least 5 amino acids, or at least 6 amino acids, or at least 7 amino acids, or at least 8 amino acids, or at least 9 amino acids, or at least 10 amino acids or more amino acids at the N-terminus, or at the C-terminus, or both at the N- and C-terminus of the corresponding amino acid sequence shown in one of above referenced sequences. Likewise, it is also contemplated that variants comprise deletions consisting of at least 1 amino acid, or at least 2 amino acids, or at least 3 amino acids, or at least 4 amino acids, or at least 5 amino acids, or at least 6 amino acids, or at least 7 amino acids, or at least 8 amino acids, or at least 9 amino acids, or at least 10 amino acids or more amino acids at the N-terminus, or at the C-terminus, or both at the N- and C-terminus of the corresponding amino acid sequence shown in one of the above mentioned sequences.

Functionally equivalent variants of a FR sequence according to the invention will preferably maintain at least 70%, at least 75%, at least 80%, at least 85%, at least 90%, at least 91 %, at least 92%, at least 93%, at least 94%, at least 95%, at least 96%, at least 97%, at least 98%, at least 99%, at least 100%, at least 105%, at least 1 10%, at least 1 15%, at least 120%, at least 125%, at least 130%, at least 135%, at least 140%, at least 145%, at least 150%, at least 200% or more of the capacity of the corresponding amino acid sequence shown in one of SEQ ID NOs: 7-13 to bind to its cognate antigen when being part of an antibody or antibody fragment of the invention. This capacity to bind to its cognate antigen may be determined as a value of affinity, avidity, specificity and/or selectivity of the antibody or antibody fragment to its cognate antigen.

In a particular embodiment, the VH region of the antibody of the invention comprises the sequence of SEQ ID NO: 15 or a functionally equivalent variant thereof and the VL region of the antibody of the invention comprises the sequence SEQ ID NO: 16 or a functionally equivalent variant thereof.

In another embodiment, the VH of the antibody of the invention comprises a sequence having at least 70%, at least 75%, at least 80%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% sequence identity with SEQ ID NO:15. In another embodiment, the VL of the antibody of the invention comprises a sequence having at least 70%, at least 75%, at least 80%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% sequence identity with SEQ ID NO:16.

Preferred embodiments of the VL and VH regions of the antibody or antibody fragment of the invention are as defined below:
1. In one embodiment, the VH of the antibody of the invention is characterized in that the CDR1 region:
   1.1. shows at least about 70%, at least about 75%, at least about 80%, at least about 85%, at least about 90%, at least about 91 %, at least about 92%, at least about 93%, at least about 94%, at least about 95%, at least about 96%, at least about 97%, at least about 98% or at least about 99% sequence identity with the sequence KASQNINKYLN (SEQ ID NO: 1), and/or
   1.2. differs in at least 1, at least 2, at least 3, at least 4, at least 5, at least 6, at least 7, at least 8, at least 9 or at least 10 amino acids with respect to the sequence of KASQNINKYLN (SEQ ID NO: 1), and/or
   1.3. at least 1, at least 2, at least 3, at least 4, at least 5, at least 6, at least 7, at least 8, at least 9, at least 10 amino acids or all amino acids are conservative substitutions of the amino acids found in the corresponding positions in the sequence KASQNINKYLN (SEQ ID NO: 1).
2. In one embodiment, the VH of the antibody of the invention is characterized in that the CDR2 region:
   2.1. shows at least about 70%, at least about 75%, at least about 80%, at least about 85%, at least about 90%, at least about 91 %, at least about 92%, at least about 93%, at least about 94%, at least about 95%, at least about 96%, at least about 97%, at least about 98% or at least about 99% sequence identity with the sequence NTNSLQT (SEQ ID NO: 2), and/or
   2.2. differs in at least 1, at least 2, at least 3, at least 4, at least 5 or at least 6 amino acids with respect to the sequence of NTNSLQT (SEQ ID NO: 2), and/or;
   2.3. at least 2, at least 3, at least 4, at least 5, at least 6 or all amino acids are conservative substitutions of the amino acids found in the corresponding positions in the sequence NTNSLQT (SEQ ID NO: 2).
3. In one embodiment the VH of the antibody of the invention is characterized in that the CDR3 region:
   3.1. shows at least about 70%, at least about 75%, at least about 80%, at least about 85%, at least about 90%, at least about 91 %, at least about 92%, at least about 93%, at least about 94%, at least about 95%, at least about 96%, at least about 97%, at least about 98% or at least about 99% sequence identity with the sequence FQHNSWPLT (SEQ ID NO: 3), and/or
   3.2. differs in at least 1, at least 2, at least 3, at least 4, at least 5, at least 6, at least 7 or at least 8 amino acids with respect to the sequence of FQHNSWPLT (SEQ ID NO: 3), and/or
   3.3. at least 1, at least 2, at least 3, at least 4, at least 5, at least 6, at least 7, at least 8 or all amino acids are conservative substitutions of the amino acids found in the corresponding positions in the sequence FQHNSWPLT (SEQ ID NO: 3).
4. In one embodiment, the VL of the antibody of the invention is characterized in that the CDR1 region:
   4.1. shows at least about 70%, at least about 75%, at least about 80%, at least about 85%, at least about 90%, at least about 91 %, at least about 92%, at least about 93%, at least about 94%, at least about 95%, at least about 96%, at least about 97%, at least about 98% or at least about 99% sequence identity with the sequence DYSVH (SEQ ID NO: 4), and/or
   4.2. differs in at least 1, at least 2, at least 3 or at least 4 amino acids with respect to the sequence of DYSVH (SEQ ID NO: 4), and/or,
   4.3. at least 1, at least 2, at least 3, at least 4 or all amino acids are conservative substitutions of the amino acids found in the corresponding positions in the sequence DYSVH (SEQ ID NO: 4).
5. In one embodiment, the VL of the antibody of the invention is characterized in that the CDR2 region:
   5.1. shows at least about 70%, at least about 75%, at least about 80%, at least about 85%, at least about 90%, at least about 91 %, at least about 92%, at least about 93%, at least about 94%, at least about 95%, at least about 96%, at least about 97%, at least about 98% or at least about 99% sequence identity with the sequence RMKFDGDTHYNSAL (SEQ ID NO: 5), and/or
   5.2. differs in at least 1, at least 2, at least 3, at least 4, at least 5, at least 6, at least 7, at least 8, at least 9, at least 10, at least 11, at least 12 or at least 13 with respect to the sequence of RMKFDGDTHYNSAL (SEQ ID NO: 5), and/or
   5.3. at least 1, at least 2, at least 3, at least 4, at least 5, at least 6, at least 7, at least 8, at least 9, at least 10, at least 11, at least 12, at least 13 or all amino acids are conservative substitutions of the amino acids found in the corresponding positions in the sequence RMKFDGDTHYNSAL (SEQ ID NO: 5).
6. In one embodiment, the VL of the antibody of the invention is characterized in that the CDR3 region:
   6.1. it shows at least about 70%, at least about 75%, at least about 80%, at least about 85%, at least about 90%, at least about 91 %, at least about 92%, at least about 93%, at least about 94%, at least about 95%, at least about 96%, at least about 97%, at least about 98% or at least about 99% sequence identity with the sequence EGYYDGTWGWY (SEQ ID NO: 6), and/or
   6.2. differs in at least 1, at least 2, at least 3, at least 4 or at least 5, at least 6, at least 7, at least 8, at least 9 or at least 10 amino acids with respect to the sequence of EGYYDGTWGWY (SEQ ID NO: 6), and/or
   6.3. at least 1, at least 2, at least 3, at least 4 or at least 5, at least 6, at least 7, at least 8, at least 9, at least 10 or all amino acids are conservative substitutions of the amino acids found in the corresponding positions in the sequence EGYYDGTWGWY (SEQ ID NO: 6).

In a particular embodiment, the antibody is humanized.

As used herein, "humanized" forms of non-human (e.g., murine) antibodies or antigen-binding domains are chimeric antibodies or antigen-binding domains that contain minimal sequence, or no sequence, derived from non-human immunoglobulin. For the most part, humanized antibodies or antigen-binding domains are human immunoglobulins (recipient antibody) in which residues from a hypervariable region of the recipient are replaced by residues from a hypervariable region of a non-human species (donor antibody) such as mouse, rat, rabbit or nonhuman primate having the desired specificity, affinity, and capacity. In some instances, Fv framework region (FR) residues of the human immunoglobulin are replaced by corresponding non-human residues. Furthermore, humanized antibodies or antigen-binding domains can comprise residues that are not found in the recipient antibody or in the donor antibody. These modifications are generally made to further refine antibody or antigen-binding domain performance. In general, the humanized antibody or antigen-binding domain will comprise substantially all of at least one, and typically two, variable domains, in which all or substantially all of the hypervariable loops correspond to those of a nonhuman immunoglobulin and all or substantially all of the FR residues are those of a human immunoglobulin sequence. The humanized antibody can also comprise at least a portion of an immunoglobulin constant region (Fc), typically that of a human immunoglobulin.

In a preferred embodiment, the antibody of the invention comprises at least one humanized framework region.

In another embodiment, the VH region of the antibody of the invention comprises at least one humanized FR region, at least 2 humanized FR regions, at least 3 humanized FR regions or at least 4 humanized FR regions, and wherein said FR regions are selected from SEQ ID NOs: 7, 8, 9 and 10.

In another embodiment, the VL region of the antibody of the invention comprises at least one humanized FR region, at least 2 humanized FR regions, at least 3 humanized FR regions or at least 4 humanized FR regions, and wherein said FR regions are selected from SEQ ID NOs: 11, 12, 13 and 14.

In another embodiment, the antibody of the invention is a Fab, a F(ab)₂, a single-domain antibody, a single chain variable fragment (scFv), or a nanobody.

Fab, F(ab)2, single-domain antibodies, single chain variable fragments (scFv), and nanobodies are considered epitope-binding antibody fragments.

An antibody fragment is a fragment of an antibody such as, for example, Fab, F(ab')2, Fab' and scFv. Various techniques have been developed for the production of antibody fragments. Traditionally, these fragments were derived via proteolytic digestion of intact antibodies but more recently these fragments can be produced directly by recombinant host cells. In other embodiments, the antibody of choice is a single chain Fv (scFv) fragment which additionally may be monospecific or bispecific.

Papain digestion of antibodies produces two identical antigen-binding fragments, called "Fab" fragments, each with a single antigen-binding site, and a residual "Fc" fragment, which name reflects its ability to crystallize readily. Pepsin treatment yields an F(ab')2 fragment that has two antigen-binding sites and is still capable of cross-linking antigen.

"Fv" is the minimum antibody fragment which contains a complete antigen-recognition and antigen-binding site. This region consists of a dimer of one heavy chain and one light chain variable domain in tight, non-covalent association. It is in this configuration that the three hypervariable regions of each variable domain interact to define an antigen-binding site on the surface of the VH-VL dimer. Collectively, the six hypervariable regions confer antigen-binding specificity to the antibody. However, even a single variable domain (or half of an Fv comprising only three hypervariable regions specific for an antigen) has the ability to recognize and bind the antigen, although with lower affinity than the entire binding site.

The Fab fragment also contains the constant domain of the light chain and the first constant domain (CHI) of the heavy chain. Fab' fragments differ from Fab fragments by the addition of a few residues at the carboxy terminus of the heavy chain CHI domain including one or more cysteines from the antibody hinge region. Fab'-SH is the designation herein for Fab' in which the cysteine residue(s) of the constant domains bear at least one free thiol group. F(ab')Z antibody fragments originally were produced as pairs of Fab' fragments which have hinge cysteines between them. Other chemical couplings of antibody fragments are also known.

The term "single domain antibodies" makes reference to antibodies whose complementary determining regions are part of a single domain polypeptide. Examples include, but are not limited to, heavy chain antibodies (nanobodies), antibodies naturally devoid of light chains, single domain antibodies derived from conventional 4-chain antibodies, engineered antibodies and single domain scaffolds other than those derived from antibodies. Single domain antibodies may be any of the art, or any future single domain antibodies. Single domain antibodies may be derived from any species including, but not limited to mouse, human, camel, llama, goat, rabbit, bovine.

"Single-chain Fv" or "scFv" antibody fragments comprise the VH and VL domains of an antibody, wherein these domains are present in a single polypeptide chain. Preferably, the Fv polypeptide further comprises a polypeptide linker between the VH and VL domains which enables the scFv to form the desired structure for antigen binding. For a review of scFv see Pluckthun in The Pharmacology of Monoclonal Antibodies, vol. 113, Rosenburg and Moore eds., Springer- Verlag, N.Y., pp. 269-315 (1994).

More preferably, although the two domains of the Fv fragment, VL and VH, are naturally encoded by separate genes, or polynucleotides that encode such gene sequences (e.g., their encoding cDNA) can be joined, using recombinant methods, by a flexible linker that enables them to be made as a single protein chain in which the VL and VH regions associate to form monovalent epitope-binding molecules (known as single-chain Fv (scFv). Alternatively, by employing a flexible linker that is too short (e.g., less than about 9 residues) to enable the VL and VH domains of a single polypeptide chain to associate together, one can form a bispecific antibody, diabody, or similar molecule (in which two such polypeptide chains associate together to form a bivalent epitope-binding molecule. Examples of epitope-binding antibody fragments encompassed within the present invention include (i) a Fab' or Fab fragment, a monovalent fragment consisting of the VL, VH, CL and CH1 domains, or a monovalent antibody as described in WO2007059782; (ii) F(ab')2 fragments, bivalent fragments comprising two Fab fragments linked by a disulfide bridge at the hinge domain; (iii) an Fd fragment consisting essentially of the VH and CH1 domains; (iv) a Fv fragment consisting essentially of a VL and VH domains, (v) a dAb fragment, which consists essentially of a VH domain and also called domain antibodies; (vi) camelid or nanobodies and (vii) an isolated complementarity determining region (CDR). Furthermore, although the two domains of the Fv fragment, VL and VH, are coded for by separate genes, they may be joined, using recombinant methods, by a synthetic linker that enables them to be made as a single protein chain in which the VL and VH domains pair to form monovalent molecules (known as single chain antibodies or single chain Fv (scFv).

The term "nanobodies" designates small sized entities (15 kDa) formed solely by the antigen binding region of the heavy chain (VH fragment) of immunoglobulins. Said nanobodies are mainly produced after immunizing animals of the Camelidae family, such as camels, llamas and dromedaries, mainly llamas; and also, of the shark family, which have the particularity of having antibodies which naturally lack the light chain and recognize the antigen by the heavy chain variable domain. Nevertheless, the nanobodies derived from these sources require a humanization process for their therapeutic application. Another potential source for obtaining nanobodies is from antibodies derived from different human samples by separating the VH and VL domains of the variable region. Nanobodies present advantages such as a production cost reduction with respect to whole antibodies, stability and the reduction of immunogenicity.

In another embodiment, the antibody of the invention is a diabody.

The term "diabodies" refers to small antibody fragments with two antigen-binding sites, those fragments comprising a heavy-chain variable domain (V_{H}) connected to a light chain variable domain (V_{L}) in the same polypeptide chain (V_{H}-V_{L}). By using a linker that is too short to allow pairing between the two domains on the same chain, the domains are forced to pair with the complementary domains of another chain and create two antigen-binding sites.

Functional fragments of antibodies which bind to the IL11RA extracellular domain included within the present invention retain at least one binding function and/or modulation function of the full-length antibody from which they are derived. Preferred functional fragments retain an antigen-binding function of a corresponding full-length antibody (e.g., the ability to bind a mammalian CCR9).

The invention may also include bispecific antibodies. Bispecific antibodies are antibodies that have binding specificities for at least two different epitopes. Exemplary bispecific antibodies may bind to two different epitopes of the IL11RA extracellular domain. Bispecific antibodies can be prepared as full-length antibodies or antibody fragments (e.g. F(ab)₂ bispecific antibodies, minibodies, diabodies). According to a different approach, antibody variable domains with the desired binding specificities (antibody-antigen combining sites) are fused to immunoglobulin constant domain sequences. The fusion preferably is with an immunoglobulin heavy chain constant domain, comprising at least part of the hinge, CH2, and CH3 regions. It is preferred to have the first heavy-chain constant region (CHI) containing the site necessary for light chain binding, present in at least one of the fusions. DNAs encoding the immunoglobulin heavy chain fusions and, if desired, the immunoglobulin light chain, are inserted into separate expression vectors, and are co-transfected into a suitable host organism. This provides great flexibility in adjusting the mutual proportions of the three polypeptide fragments in embodiments when unequal ratios of the three polypeptide chains used in the construction provide the optimum yields. It is, however, possible to insert the coding sequences for two or all three polypeptide chains in one expression vector when the expression of at least two polypeptide chains in equal ratios results in high yields or when the ratios are of no particular significance.

Techniques for generating bispecific antibodies from antibody fragments have also been described in the literature. For example, bispecific antibodies can be prepared using chemical linkage.

The fragments with the capacity to bind to the IL11RA extracellular domain can also be obtained by conventional methods known by persons having ordinary skill in the art. Said methods can involve isolating DNA that encodes the polypeptide chains (or a fragment thereof) of a monoclonal antibody of interest and manipulating DNA by means of recombinant DNA technology. DNA can be used to generate another DNA of interest, or an altered DNA, (for example by means of mutagenesis) for adding, removing or substituting one or more amino acids, for example, the DNA that encodes the polypeptide chains of an antibody (e.g., the heavy or light chains, the variable region or the whole antibody) can be isolated from murine B cells from immunized mice with the IL11RA extracellular domain. The DNA can be isolated and amplified by conventional methods, for example by means of PCR.

The single-chain antibodies can be obtained by conventional methods by binding the variable region of the heavy and light chains (Fv region) by means of an amino acid bridge. The scFvs can be prepared by fusing the DNA encoding a linker peptide between the DNAs encoding the polypeptides of the variable regions (V_{L} and V_{H}). The production of scFvs is described in a number of documents, for example, in US patent US 4,946,778, Bird (Science 242: 423, 1988), Huston et al. (Proc. Natl. Acad Sci USA 85: 5879, 1988) and Ward et al. (Nature 334: 544, 1989).

In a further embodiment, the antibody of the invention is coupled to a detectable label. In another preferred embodiment, said label can be detected by means of a change in at least one of its physical, chemical, electrical or magnetic properties.

In the context of the present invention, the term "detectable label" or "labelling agent", as used herein, refers to a molecular label which allows the detection, localization and/or identification of the molecule to which it is attached, using suitable procedures and equipment for detection, for example by spectroscopic, photochemical, biochemical, immunochemical or chemical means. Labelling agents that are suitable for labelling the antibodies include radionuclides, enzymes, fluorophores, chemiluminescent reagents, enzyme substrates or cofactors, enzyme inhibitors, particles, magnetic particles, dyes and derivatives, and the like.

The compounds radioactively labeled by means of radioactive isotopes, also called radioisotopes or radionuclides, may include, without limitation, 3H, 14C, 15N, 35S, 90Y, 99Tc, mln, 1251, 1311, 133Xe, 111Lu, 211At and 213 B. Radioisotope labelling is performed typically by using chelating ligands that are capable of complexing metal ions such as DOTA, DOTP, DOTMA, DTPA and TETA. Methods for conjugating radioisotopes to proteins are well known in the prior art.

In another particular embodiment, the antibody of the invention is labelled with a fluorescent group. The fluorescent group can be attached to the side chains of the amino acids directly or through a linking group. Methods for conjugating polypeptides fluorescent reagents are well known in the prior art.

Suitable reagents for labelling polypeptides, such as antibodies, with fluorescent groups include chemical groups which show ability to react with the various groups listed in the side chains of the proteins, including amino groups and thiol groups. Thus, chemical groups that can be used to modify the antibodies according to the present invention include, without limitation, maleimide, haloacetyl, iodoacetamide succinimidyl ester (e.g. NHS, N-hydroxysuccinimide), isothiocyanate, sulfonyl chloride, 2,6-dichlorotriazinyl, pentafluorophenyl ester, phosphoramidite and the like. An example of suitable reactive functional group is N -hydroxysuccinimide ester (NHS) of a detectable group modified with a carboxyl group. Typically , the carboxyl group modifying the fluorescent compound is activated by the contacting of said compound with a carbodiimide reagent (for example, dicyclohexylcarbodiimide, diisopropylcarbodiimide, uranium or a reagent such as TSTU (0-(N-Succinimidyl)-N,N,N',N'-tetramethyluronium tetrafluoroborate), HBTU((0-benzotriazol- 1 -yl)-N,N,N',N'-tetramethyluronium hexafluorophosphate), or HATU (0-(7-azabenzotriazol-I-yl)-N,N,N',N'-tetramethyluronium hexafluorophosphate), an activator of the type of 1-hydroxybenzotriazole (HOBt) and N-hydroxysuccinimide to give the NHS ester of the label.

The fluorescent labels may include, without limitation, ethidium bromide, SYBR Green, fluorescein isothiocyanate (FITC), rhodamine tetramethyl isotiol (TRIT), 5-carboxyfluorescein, 6-carboxyfluorescein, fluorescein, HEX (6-carboxy-2',4,4',5',7,7'-hexachlorofluorescein), Oregon Green 488, Oregon Green 500, Oregon Green 514, Joe (6-carboxy-4',5'-dichloro-2',7'-dimethoxyfluorescein), 5-carboxy-2',4',5',7'-tetrachlorofluorescein,5-carboxyrhodamine, rhodamine, tetramethylrhodamine (Tamra), Rox (carboxy-X-rhodamine), R6G (rhodamine 6G), phthalocyanines, azometazinas, cyanines (Cy2,Cy3 and Cy5), Texas Red, Princeston Red, BODIPY FL-Br2, BODIPY 530/550, BODIPY TMR, BODIPY 558/568, BODIPY 564/570, BODIPY 576/589, BODIPY 581/591, BODIPY TR, BODIPY 630/650, BODIPY 650/665, DABCYL, eosin, erythrosin, ethidium bromide, green fluorescent protein (GFP) and its analogues, inorganic-based fluorescent semiconductor nanocrystals (Quantum Dot), fluorescent labels based onlanthanide such as Eu3+ and Sm3+ and the like, rhodamine, phosphorus-lanthanides or FITC.

The enzymatic labels may include, without limitation, horseradish peroxidase, β-galactosidase, luciferase or alkaline phosphatase.

In another particular embodiment, the antibody of the invention is labelled by conjugation to a first member of a binding pair. In a preferred embodiment, this modification is covalent biotinylation. The term "biotinylation", as used herein, refers to the covalent attachment of biotin to a molecule (typically a protein). Biotinylation is performed using biotin reagents capable of conjugating to the side chain of the proteins, wherein said conjugation occurs primarily on the primary amino groups and thiol groups contained in the side chains of proteins. Suitable reagents for biotinylation of amino groups include molecules containing biotin and a group capable of reacting with amino groups such as succinimide esters, pentafluorophenyl ester or alkyl halides, wherein the biotin moiety and the reactive group separated by a spacer of any length.

In another particular embodiment, the antibody of the invention is labelled with metal ions such as gold (Au), including colloidal gold nanoparticles can be attached directly to the antibody via electrostatic interactions. In another particular embodiment, the colloidal gold nanoparticles are pre-coupled to biotin and can be covalently attached to the antibody.

### Nucleic acids and host cells related to the antibody of the invention

In a second aspect, the invention relates to a nucleic acid encoding the antibody of the invention.

The term "nucleic acid" or "polynucleotide" refers to deoxyribonucleic acids (DNA) or ribonucleic acids (RNA) and their polymers in either single or double stranded form. Unless specifically limited, the term encompasses nucleic acids containing known analogs of natural nucleotides that have binding capabilities similar to those of a reference nucleic acid and which are metabolized similarly to naturally occurring nucleotides. Unless otherwise indicated, a specific nucleic acid sequence also implies conservatively modified variants (e.g., substitutions with degenerate codons), alleles, orthologs, SNPs and complementary sequences, as well as sequences indicated in direct form. In particular, substitutions with degenerate codons can be obtained by creating sequences in which the third position of one or more selected (or all) codons is replaced by residues with mixed bases and/or deoxyinosine residues.

Exemplary nucleic acids or polynucleotides of the invention include, but are not limited to, ribonucleic acids (RNAs), deoxyribonucleic acids (DNAs), threose nucleic acids (TNAs), glycol nucleic acids (GNAs), peptide nucleic acids (PNAs), locked nucleic acids (LNAs, including LNA having a β-D-ribo configuration, a-LNA having an a-L- ribo configuration (a diastereomer of LNA), 2'-amino-LNA having a 2 '-amino functionalization, and 2'-amino- a-LNA having a 2'-amino functionalization), ethylene nucleic acids (ENA), cyclohexenyl nucleic acids (CeNA) or hybrids or combinations thereof.

In some cases, a subject nucleic acid provides for production of he antibody of the present disclosure, e.g., in a mammalian cell. In other cases, a subject nucleic acid provides for amplification of the antibody-encoding nucleic acid.

A nucleotide sequence encoding he antibody of the present invention can be operably linked to a transcriptional control element, e.g., a promoter, and enhancer, etc.

Suitable promoter and enhancer elements are known in the art. For expression in a bacterial cell, suitable promoters include, but are not limited to, lacI, lacZ, T3, T7, gpt, lambda P and trc. For expression in a eukaryotic cell, suitable promoters include, but are not limited to, light and/or heavy chain immunoglobulin gene promoter and enhancer elements; cytomegalovirus immediate early promoter; herpes simplex virus thymidine kinase promoter; early and late SV40 promoters; promoter present in long terminal repeats from a retrovirus; mouse metallothionein-I promoter; and various art-known tissue specific promoters.

Suitable reversible promoters, including reversible inducible promoters are known in the art. Such reversible promoters may be isolated and derived from many organisms, e.g., eukaryotes and prokaryotes. Modification of reversible promoters derived from a first organism for use in a second organism, e.g., a first prokaryote and a second a eukaryote, a first eukaryote and a second a prokaryote, etc., is well known in the art. Such reversible promoters, and systems based on such reversible promoters but also comprising additional control proteins, include, but are not limited to, alcohol regulated promoters (e.g., alcohol dehydrogenase I (alcA) gene promoter, promoters responsive to alcohol transactivator proteins (AlcR), etc.), tetracycline regulated promoters, (e.g., promoter systems including TetActivators, TetON, TetOFF, etc.), steroid regulated promoters (e.g., rat glucocorticoid receptor promoter systems, human estrogen receptor promoter systems, retinoid promoter systems, thyroid promoter systems, ecdysone promoter systems, mifepristone promoter systems, etc.), metal regulated promoters (e.g., metallothionein promoter systems, etc.), pathogenesis-related regulated promoters (e.g., salicylic acid regulated promoters, ethylene regulated promoters, benzothiadiazole regulated promoters, etc.), temperature regulated promoters (e.g., heat shock inducible promoters (e.g., HSP-70, HSP-90, soybean heat shock promoter, etc.), light regulated promoters, synthetic inducible promoters, and the like.

In some instances, the locus or construct or transgene containing the suitable promoter is irreversibly switched through the induction of an inducible system. Suitable systems for induction of an irreversible switch are well known in the art, e.g., induction of an irreversible switch may make use of a Cre-Iox-mediated recombination. Any suitable combination of recombinase, endonuclease, ligase, recombination sites, etc. known to the art may be used in generating an irreversibly switchable promoter. Methods, mechanisms, and requirements for performing site-specific recombination, described elsewhere herein, find use in generating irreversibly switched promoters and are well known in the art.

In some cases, the promoter is a CD8 cell-specific promoter, a CD4 cell-specific promoter, a neutrophil-specific promoter, or an NK-specific promoter. For example, a CD4 gene promoter can be used. As another example, a CD8 gene promoter can be used. NK cell-specific expression can be achieved by use of an Ncr1 (p46) promoter; see, e.g., Eckelhart et al. (2011) Blood 117:1565.

In some embodiments, e.g., for expression in a yeast cell, a suitable promoter is a constitutive promoter such as an ADH1 promoter, a PGK1 promoter, an ENO promoter, a PYK1 promoter and the like; or a regulatable promoter such as a GAL1 promoter, a GAL10 promoter, an ADH2 promoter, a PHO5 promoter, a CUP1 promoter, a GAL7 promoter, a MET25 promoter, a MET3 promoter, a CYC1 promoter, a HIS3 promoter, an ADH1 promoter, a PGK promoter, a GAPDH promoter, an ADC1 promoter, a TRP1 promoter, a URA3 promoter, a LEU2 promoter, an ENO promoter, a TP1 promoter, and AOX1 (e.g., for use in Pichia). Selection of the appropriate vector and promoter is well within the level of ordinary skill in the art.

Suitable promoters for use in prokaryotic host cells include, but are not limited to, a bacteriophage T7 RNA polymerase promoter; a trp promoter; a lac operon promoter; a hybrid promoter, e.g., a lac/tac hybrid promoter, a tac/trc hybrid promoter, a trp/lac promoter, a T7/lac promoter; a trc promoter; a tac promoter, and the like; an araBAD promoter; in vivo regulated promoters, such as an ssaG promoter or a related promoter, a pagC promoter, a nirB promoter, and the like; a sigma70 promoter, e.g., a consensus sigma70 promoter; a stationary phase promoter, e.g., a dps promoter, an spv promoter, and the like; a promoter derived from the pathogenicity island SPI-2; an actA promoter; an rpsM promoter; a tet promoter; an SP6 promoter; and the like. Suitable strong promoters for use in prokaryotes such as Escherichia coli include, but are not limited to Trc, Tac, T5, T7, and P Lambda. Non-limiting examples of operators for use in bacterial host cells include a lactose promoter operator (LacI repressor protein changes conformation when contacted with lactose, thereby preventing the LacI repressor protein from binding to the operator), a tryptophan promoter operator (when complexed with tryptophan, TrpR repressor protein has a conformation that binds the operator; in the absence of tryptophan, the TrpR repressor protein has a conformation that does not bind to the operator), and a tac promoter operator.

Any of the polynucleotides described above may further include additional nucleic acids, encoding, e.g. a signal peptide to direct secretion of the encoded antibody constant regions as described herein. Also, as described in more detail elsewhere herein, the present invention includes compositions comprising one or more of the polynucleotides described above.

In one embodiment, the invention includes compositions comprising a first polynucleotide and second polynucleotide wherein said first polynucleotide encodes a VH domain as described herein and wherein said second polynucleotide encodes a VL domain as described herein.

The present invention also includes fragments of the polynucleotides of the invention. Additionally, polynucleotides that encode fusion polypeptides, Fab fragments, and other derivatives, as described herein, are also contemplated by the invention.

The polynucleotides may be produced or manufactured by any method known in the art. For example, if the nucleotide sequence of the antibody is known, a polynucleotide encoding the antibody may be assembled from chemically synthesized oligonucleotides (e.g., as described in Kutmeier et al., Bio Techniques 17:242 (1994)), which, briefly, involves the synthesis of overlapping oligonucleotides containing portions of the sequence encoding the antibody, annealing and ligating of those oligonucleotides, and then amplification of the ligated oligonucleotides by PCR.

Alternatively, a polynucleotide encoding the IL11RA extracellular domain antibody, may be generated from nucleic acid from a suitable source. If a clone containing a nucleic acid encoding a particular antibody is not available, but the sequence of the antibody molecule is known, a nucleic acid encoding the antibody may be chemically synthesized or obtained from a suitable source (e.g., an antibody cDNA library, or a cDNA library generated from, or nucleic acid, preferably poly A+RNA, isolated from, any tissue or cells expressing the antibody or other anti- IL11RA extracellular domain antibody, such as hybridoma cells selected to express an antibody by PCR amplification using synthetic primers hybridizable to the 3' and 5' ends of the sequence or by cloning using an oligonucleotide probe specific for the particular gene sequence to identify, e.g., a cDNA clone from a cDNA library that encodes the antibody. Amplified nucleic acids generated by PCR may then be cloned into replicable cloning vectors using any method well known in the art.

Once the nucleotide sequence and corresponding amino acid sequence of the anti-IL11RA extracellular domain antibody, or antigen-binding fragment, variant, or derivative thereof is determined, its nucleotide sequence may be manipulated using methods well known in the art for the manipulation of nucleotide sequences, e.g., recombinant DNA techniques, site directed mutagenesis, PCR, etc. (see, for example, the techniques described in Sambrook et al. (1990) Molecular Cloning, A Laboratory Manual (2nd ed.; Cold Spring Harbor Laboratory, Cold Spring Harbor, N.Y.) and Ausubel et al., eds. (1998) Current Protocols in Molecular Biology (John Wiley & Sons, NY), which are both incorporated by reference herein in their entireties), to generate antibodies having a different amino acid sequence, for example to create amino acid substitutions, deletions, and/or insertions.

A polynucleotide encoding an anti- IL11RA extracellular domain antibody, or antigen-binding fragment, variant, or derivative thereof, can be composed of any polyribonucleotide or polydeoxyribonucleotide, which may be unmodified RNA or DNA or modified RNA or DNA. For example, a polynucleotide encoding anti-IL11RA extracellular domain antibody, or antigen-binding fragment, variant, or derivative thereof can be composed of single- and double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single- and double-stranded regions. In addition, a polynucleotide encoding an anti-IL11RA extracellular domain antibody, or antigen-binding fragment, variant, or derivative thereof can be composed of triple-stranded regions comprising RNA or DNA or both RNA and DNA.

A polynucleotide encoding an anti-IL11RA extracellular domain antibody, or antigen-binding fragment, variant, or derivative thereof, may also contain one or more modified bases or DNA or RNA backbones modified for stability or for other reasons. "Modified" bases include, for example, tritylated bases and unusual bases such as inosine. A variety of modifications can be made to DNA and RNA; thus, "polynucleotide" embraces chemically, enzymatically, or metabolically modified forms.

An isolated polynucleotide encoding a non-natural variant of a polypeptide derived from an immunoglobulin (e.g., an immunoglobulin heavy chain portion or light chain portion) can be created by introducing one or more nucleotide substitutions, additions or deletions into the nucleotide sequence of the immunoglobulin such that one or more amino acid substitutions, additions or deletions are introduced into the encoded protein. Mutations may be introduced by standard techniques, such as site-directed mutagenesis and PCR-mediated mutagenesis. Preferably, conservative amino acid substitutions are made at one or more non-essential amino acid residues.

In one embodiment, the polynucleotides have a modular design to encode at least one of the antibodies, fragments or variants thereof described herein. As a non- limiting example, the polynucleotide construct may encode any of the following designs: (1) the heavy chain of an antibody, (2) the light chain of an antibody, (3) the heavy and light chain of the antibody, (4) the heavy chain and light chain separated by a linker, (5) the VHI, CHI, CH2, CH3 domains, a linker and the light chain and (6) the VHI, CHI, CH2, CH3 domains, VL region, and the light chain. Any of these designs may also comprise optional linkers between any domain and/or region.

In a particular embodiment the polynucleotide of the invention encodes a Fab, a F(ab)2, a single domain antibody, a single chain variable fragment (scFv), or a nanobody.

In a third aspect the invention relates to an expression vector comprising the nucleic acid of the invention.

As used herein, "vector," "cloning vector," and "expression vector" are vehicles by which the host is transformed and expression of introduced sequences (e.g., transcription and translation) Mean a vehicle in which a polynucleotide sequence (e.g., a foreign gene) can be introduced into a host cell to facilitate Vectors include plasmids, phages, viruses and the like.

A nucleotide sequence encoding the antibody of the invention can be present in an expression vector and/or a cloning vector. An expression vector can include a selectable marker, an origin of replication, and other features that provide for replication and/or maintenance of the vector. Suitable expression vectors include, e.g., plasmids, viral vectors, and the like.

Large numbers of suitable vectors and promoters are known to those of skill in the art; many are commercially available for generating a subject recombinant constructs. The following vectors are provided by way of example. Bacterial: pBs, phagescript, PsiX174, pBluescript SK, pBs KS, pNH8a, pNH16a, pNH18a, pNH46a (Stratagene, La Jolla, Calif., USA); pTrc99A, pKK223-3, pKK233-3, pDR540, and pRIT5 (Pharmacia, Uppsala, Sweden). Eukaryotic: pWLneo, pSV2cat, pOG44, PXR1, pSG (Stratagene) pSVK3, pBPV, pMSG and pSVL (Pharmacia).

Expression vectors generally have convenient restriction sites located near the promoter sequence to provide for the insertion of nucleic acid sequences encoding heterologous proteins. A selectable marker operative in the expression host may be present. Suitable expression vectors include, but are not limited to, viral vectors (e.g. viral vectors based on vaccinia virus; poliovirus; adenovirus; adeno-associated virus; SV40; herpes simplex virus; human immunodeficiency virus; a retroviral vector (e.g., Murine Leukemia Virus, spleen necrosis virus, and vectors derived from retroviruses such as Rous Sarcoma Virus, Harvey Sarcoma Virus, avian leukosis virus, human immunodeficiency virus, myeloproliferative sarcoma virus, and mammary tumor virus); and the like.

As noted above, in some embodiments, a nucleic acid comprising the antibody of the invention will in some embodiments be RNA, e.g., in vitro synthesized RNA. Methods for in vitro synthesis of RNA are known in the art; any known method can be used to synthesize RNA comprising a nucleotide sequence encoding the antibody of the invention. Methods for introducing RNA into a host cell are known in the art. Introducing RNA comprising a nucleotide sequence encoding the antibody of the invention into a host cell can be carried out in vitro or ex vivo or in vivo. For example, a host cell (e.g., an NK cell, a cytotoxic T lymphocyte, etc.) can be electroporated in vitro or ex vivo with RNA comprising a nucleotide sequence encoding the antibody of the invention.

In order to assess the expression of the antibody thereof, the expression vector to be introduced into a cell can also contain either a selectable marker gene or a reporter gene or both to facilitate identification and selection of expressing cells from the population of cells sought to be transfected or infected through viral vectors; in other aspects, the selectable marker may be carried on a separate piece of DNA and used in a co-transfection procedure. Both selectable markers and reporter genes may be flanked with appropriate regulatory sequences to enable expression in the host cells. Useful selectable markers include, for example, antibiotic -resistance genes, such as neo and the like. Reporter genes are used for identifying potentially transfected cells and for evaluating the functionality of regulatory sequences. In general, a reporter gene is a gene that is not present in or expressed by the recipient organism or tissue and that encodes a polypeptide whose expression is manifested by some easily detectable property, e.g., enzymatic activity. Expression of the reporter gene is assayed at a suitable time after the DNA has been introduced into the recipient cells. Suitable reporter genes may include genes encoding luciferase, beta-galactosidase, chloramphenicol acetyl transferase, secreted alkaline phosphatase, or the green fluorescent protein gene. Suitable expression systems are well known and may be prepared using known techniques or obtained commercially. In general, the construct with the minimal 5' flanking region showing the highest level of expression of reporter gene is identified as the promoter. Such promoter regions may be linked to a reporter gene and used to evaluate agents for the ability to modulate promoter- driven transcription.

In a fourth aspect, the invention relates to a host cell comprising the nucleic acid of the invention or the expression vector of the invention.

The term "host cell", as used herein, refers to a cell into which a nucleic acid of the invention, such as a polynucleotide or a vector according to the invention, has been introduced and is capable of expressing the micropeptides of the invention. The terms "host cell" and "recombinant host cell" are used interchangeably herein. It should be understood that such terms refer not only to the particular subject cell but to the progeny or potential progeny of such a cell. Because certain modifications may occur in succeeding generations due to either mutation or environmental influences, such progeny may not, in fact, be identical to the parent cell, but are still included within the scope of the term as used herein. The term includes any cultivatable cell that can be modified by the introduction of heterologous DNA. Preferably, a host cell is one in which the polynucleotide of the invention can be stably expressed, post-translationally modified, localized to the appropriate subcellular compartment, and made to engage the appropriate transcription machinery. The choice of an appropriate host cell will also be influenced by the choice of detection signal.

Suitable host cells for cloning or expressing the DNA in the vectors herein are the prokaryote, yeast, or higher eukaryote cells described above. Suitable prokaryotes for this purpose include eubacteria, such as Gram-negative or Gram-positive organisms, for example, Enterobacteriaceae such as *Escherichia,* e.g., *E. coli, Enterobacter, Erwinia, Klebsiella, Proteus, Salmonella,* e.g., *Salmonella typhimurium, Serratia,* e.g., *Serratia marcescans,* and *Shigella,* as well as *Bacilli* such as *B. subtilis* and *B. licheniformis* (e.g., *B. licheniformis* 41P disclosed in DD 266,710 published 12 Apr. 1989), *Pseudomonas* such as *P. aeruginosa,* and *Streptomyces.* One preferred *E*. *coli* cloning host is *E. coli* 294 (ATCC 31,446), although other strains such as *E. coli* B, *E. coli* X1776 (ATCC 31,537), and *E. coli* W31 10 (ATCC 27,325) are suitable. These examples are illustrative rather than limiting.

Full length antibody or antibody fragments can be produced in bacteria, in particular when glycosylation and Fc effector function are not needed, such as when the therapeutic antibody is conjugated to a cytotoxic agent (e.g., a toxin) and the immunoconjugate by itself shows effectiveness in tumor cell destruction. Full length antibodies have greater half-life in circulation. Production in *E. coli* is faster and more cost efficient. For expression of antibody fragments and polypeptides in bacteria, see, e.g., U.S. Pat. No. 5,648,237 (Carter et. al.), U.S. Pat. No. 5,789,199 (Joly et al.), and U.S. Pat. No. 5,840,523 (Simmons et al.) which describes translation initiation region (TIR) and signal sequences for optimizing expression and secretion, these patents incorporated herein by reference. After expression, the antibody is isolated from the *E*. *coli* cell paste in a soluble fraction and can be purified through, e.g., a protein A or G column depending on the isotype. Final purification can be carried out similar to the process for purifying antibody expressed e.g, in CHO cells.

In addition to prokaryotes, eukaryotic microbes such as filamentous fungi or yeast are suitable cloning or expression hosts for anti-glycosylated IL11RA extracellular domain-antibody-encoding vectors. *Saccharomyces cerevisiae,* or common baker's yeast, is the most commonly used among lower eukaryotic host microorganisms. However, a number of other genera, species, and strains are commonly available and useful herein, such as *Schizosaccharomyces pombe; Kluyveromyces* hosts such as, e.g., *K. lactis, K. fragilis* (ATCC 12,424), *K. bulgaricus* (ATCC 16,045), *K. wickeramii* (ATCC 24,178), *K. waltii* (ATCC 56,500), *K. drosophilarum* (ATCC 36,906), *K. thermotolerans,* and *K*. *marxianus; yarrowia* (EP 402,226); *Pichia pastoris* (EP 183,070); *Candida; Trichoderma reesia* (EP 244,234); *Neurospora crassa; Schwanniomyces* such as *Schwanniomyces occidentalis;* and filamentous fungi such as, e.g., *Neurospora, Penicillium, Tolypocladium,* and *Aspergillus* hosts such as *A. nidulans* and *A. niger.*

Suitable host cells for the expression of anti- IL11RA extracellular domain antibody are derived from multicellular organisms. Examples of invertebrate cells include plant and insect cells. Numerous baculoviral strains and variants and corresponding permissive insect host cells from hosts such as *Spodoptera frugiperda* (caterpillar), *Aedes aegypti* (mosquito), *Aedes albopictus* (mosquito), *Drosophila melanogaster* (fruitfly), and *Bombyx mori* have been identified. A variety of viral strains for transfection are publicly available, e.g., the L-1 variant of *Autographa californica* NPV and the Bm-5 strain of *Bombyx mori* NPV, and such viruses may be used as the virus herein according to the present invention, particularly for transfection of *Spodoptera frugiperda* cells.

Plant cell cultures of cotton, corn, potato, soybean, petunia, tomato, *Arabidopsis* and tobacco can also be utilized as hosts. Cloning and expression vectors useful in the production of proteins in plant cell culture are known to those of skill in the art. See e.g. Hiatt et al., Nature (1989) 342: 76-78, Owen et al. (1992) Bio/Technology 10: 790-794, Artsaenko et al. (1995) The Plant J 8: 745-750, and Fecker et al. (1996) Plant Mol Biol 32: 979-986.

However, interest has been greatest in vertebrate cells, and propagation of vertebrate cells in culture (tissue culture) has become a routine procedure. Examples of useful mammalian host cell lines are monkey kidney CVI line transformed by SV40 (COS-7, ATCC CRL 1651); human embryonic kidney line (293 or 293 cells subcloned for growth in suspension culture, Graham et al., J. Gen Virol. 36:59 (1977)); baby hamster kidney cells (BHK, ATCC CCL 10); Chinese hamster ovary cells/-DHFR (CHO, Urlaub et al., Proc. Natl. Acad. Sci. USA 77:4216 (1980)); mouse sertoli cells (TM4, Mather, Biol. Reprod. 23:243-251 (1980)); monkey kidney cells (CVI ATCC CCL 70); African green monkey kidney cells (VERO-76, ATCC CRL1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, 1413 8065); mouse mammary tumor (MMT 060562, ATCC CCL5 1); TRI cells (Mather et al., Annals N.Y. Acad. Sci. 383:44-68 (1982)); MRC 5 cells; FS4 cells; and a human hepatoma line (Hep G2).

In an embodiment, the cell comprising the antibody described herein; or a nucleic acid encoding the antibody described herein is a mammalian cell.

Suitable mammalian cells include primary cells and immortalized cell lines. Suitable mammalian cell lines include human cell lines, non-human primate cell lines, rodent (e.g., mouse, rat) cell lines, and the like. Suitable mammalian cell lines include, but are not limited to, HeLa cells (e.g., American Type Culture Collection (ATCC) No. CCL-2), CHO cells (e.g., ATCC Nos. CRL9618, CCL61, CRL9096), 293 cells (e.g., ATCC No. CRL-1573), Vero cells, NIH 3T3 cells (e.g., ATCC No. CRL-1658), Huh-7 cells, BHK cells (e.g., ATCC No. CCL10), PC12 cells (ATCC No. CRL1721), COS cells, COS-7 cells (ATCC No. CRL1651), RAT1 cells, mouse L cells (ATCC No. CCLI.3), human embryonic kidney (HEK) cells (ATCC No. CRL1573), HLHepG2 cells, Hut-78, Jurkat, HL-60, NK cell lines (e.g., NKL, NK92, and YTS), and the like.

In one embodiment, the mammalian cell comprises the antibody described herein. The mammalian cell or tissue can be of human, primate, hamster, rabbit, rodent, cow, pig, sheep, horse, goat, dog or cat origin, but any other mammalian cell may be used. In a preferred embodiment of any aspect, the mammalian cell is human.

In some instances, the cell is not an immortalized cell line, but is instead a cell (e.g., a primary cell) obtained from an individual. For example, in some cases, the cell is an immune cell obtained from an individual.

In another embodiment, the engineered cells may be obtained from peripheral blood, cord blood, bone marrow, tumor infiltrating lymphocytes, lymph node tissue, or thymus tissue. The host cells may include placental cells, embryonic stem cells, induced pluripotent stem cells, or hematopoietic stem cells. The cells may be obtained from humans, monkeys, chimpanzees, dogs, cats, mice, rats, and transgenic species thereof. The cells may be obtained from established cell lines.

Host cells are transformed with the above-described expression or cloning vectors for anti-IL11RA extracellular domain antibody production and cultured in conventional nutrient media modified as appropriate for inducing promoters, selecting transformants, or amplifying the genes encoding the desired sequences.

### Antibody-drug conjugates (ADCs)

In a further aspect, the antibody of the invention is conjugated with at least one therapeutic agent, which it may be a radioisotope, radionuclide, toxin, toxoid, a polypeptide or chemotherapeutic agent. When an ScFv, antigen-binding domain or antibody is conjugated to a drug it is referred to as an antibody-drug conjugates (ADCs) or, when an ScFv, antigen-binding domain or antibody are conjugated to a toxin, they are called immunotoxins. Both, ADCs and immunotoxins are included within the scope of the present invention. When administered to a patient, ADCs and immunotoxins bind to the target cell via their antibody portions and are internalized allowing drugs or toxins to exert their effects.

Thus, in a fifth aspect, the invention relates to an antibody-drug conjugate (ADC) comprising:
(i) a therapeutic agent, and
(ii) an antibody according to the invention.

The terms "therapeutic agent" or "drug" refer to refers to a pharmacologically active substance or substances that are used to treat, or prevent diseases or conditions. Drugs act by altering the physiology of a living organism, tissue, cell, or in vitro system that they are exposed to. In a particular embodiment, the therapeutic agent is a cytotoxic agent. As mentioned above, the term "antibody-drug conjugate" or "ADC" refers to antibodies, or antigen-binding domains as the ones previously defined within the context of the CARs and the antigen-binding domains and antibodies of the invention, also including antibody derivatives, that bind to the IL11RA extracellular domain and are conjugated to a drug such as a cytotoxic, cytostatic, and/or therapeutic agent.

Therefore, the antibody conjugates of the invention relate to an antibody or antibody fragment thereof recombinantly fused or chemically conjugated (covalent bonds and chemically) to heterologous agents to create fusion proteins. The heterologous agent is a polypeptide (or portion thereof, preferably a polypeptide of at least 10, at least 20, at least 30, at least 40, at least 50, at least 60, at least 70, at least 80, at least 90 or at least 100 amino acids), nucleic acid, small It may be a molecule (less than 1000 daltons) or an inorganic or organic compound. The fusion need not be direct and may occur through a linker sequence.

In an embodiment the antibody according to the invention forming part of the ADC is humanized.

In another embodiment, the antibody of the invention forming part of the ADC comprises at least one humanized framework region.

In another embodiment, the VH region of the antibody of the ADC comprises at least one humanized FR region, at least 2 humanized FR regions, at least 3 humanized FR regions or at least 4 humanized FR regions, and wherein said FR regions are selected from SEQ ID NOs: 7, 8, 9 and 10.

In another embodiment, the VL region of the antibody of the ADC comprises at least one humanized FR region, at least 2 humanized FR regions, at least 3 humanized FR regions or at least 4 humanized FR regions, and wherein said FR regions are selected from SEQ ID NOs: 11, 12, 13 and 14.

In another embodiment, the antibody of the ADC is a Fab, a F(ab)2, a single-domain antibody, a single chain variable fragment (scFv), or a nanobody.

The above related terms have already been described within the context of the antibody of the invention and are equally applicable to the present case.

In a particular embodiment, the therapeutic agent is a cytotoxic agent.

The term "cytotoxic agent" as used herein refers to a substance that inhibits or prevents the function of cells and/or causes destruction of cells. The term is intended to include radioactive isotopes (e.g., At, I, Y, Re, Re, Sm, TBi, ∼P, C, and radioactive isotopes of Lu), chemotherapeutic agents; and toxins such as small molecule toxins or enzymatically active toxins of bacterial, fungal, plant or animal origin, including synthetic analogs and derivatives thereof.

In another particular embodiment, the cytotoxic agent is a chemotherapeutic agent, or an agent for targeted therapy or immunotherapy.

A "chemotherapeutic agent" is a chemical agent (e.g., compound or drug) useful in the treatment of cancer, regardless of mechanism of action. Classes of chemotherapeutic agents include, but are not limited to: alkylating agents, antimetabolites, spindle poison plant alkaloids, cytotoxic/antitumor antibiotics, topoisomerase inhibitors, antibodies, photosensitizers, and kinase inhibitors.

"Targeted cancer therapies" are drugs or other substances that block the growth and spread of cancer by interfering with specific molecules ("molecular targets") that are involved in the growth, progression, and spread of cancer. Targeted cancer therapies are sometimes called "molecularly targeted drugs," "molecularly targeted therapies," "precision medicines," or similar names. Many different targeted therapies have been approved for use in cancer treatment. These therapies include hormone therapies, signal transduction inhibitors, gene expression modulators, apoptosis inducers, angiogenesis inhibitors, immunotherapies, and toxin delivery molecules.

Examples of targeted therapy and chemotherapeutic agents include, but are not limited to, Erlotinib (TARCEVA^{®}, Genentech/OSI Pharm.), Bortezomib (VELCADE^{®}, Millenium Pharm.), Fulvestrant (FASLODEX^{®}, Astrazeneca), Sutent (SU11248, Pfizer), Letrozole (FEMARA^{®}, Novartis), Imatinib mesylate (GLEEVEC^{®}, Novartis), PTK787/Z 222584 (Novartis), Oxaliplatin (Eloxatin^{®}, Sanofi), 5-FU (5-fluorouracil), Leucovorin, Rapamycin (Sirolimus, RAPAMUNE^{®}, Wyeth), Lapatinib (GSK572016, GlaxoSmithKline), Lonafarnib (SCH 66336), Sorafenib (BAY43-9006, Bayer Labs.), and Gefitinib (IRESSA^{®}, Astrazeneca), AG1478, AG1571 (SU 5271; Sugen), alkylating agents such as thiotepa and CYTOXAN^{®} cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, triethylenephosphoramide, triethylenethiophosphorarnide and trimethylomelarnine; acetogenins (especially bullatacin and bullatacinone); a camptothecin (including the synthetic analogue topotecan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, -2189 and CB1-TM1); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; antibiotics such as the enediyne antibiotics (e.g calicheamicin, especially calicheamicin gammall and calicheamicin omegall (Angew Chem Intl. Ed. Engl. (1994) 33:183-186); dynemicin, including dynemicin A; bisphosphonates, such as clodronate; an esperamicin; as well as neocarzinostatin chromophore and related chromoprotein enediyne antibiotic chromophores), aclacinoraysins, actinomycin, authramycin, azaserine, bleomycins, cactinomycin, carabicin, canninomycin, carzinophilin, chromomycinis, dactinomycin, daunorubicin, detorubicin, 6-diazo-5-oxo-L-norleucine, ADRIAMYCIN^{®} doxorubicin (including morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptpnigrin, streptozocin, tubercidin, ubenimex, zinostatin, zorubicin; antimetabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti- adrenals such as anunoglutetWmide, mitotane, trilostane; folic acid replenisher such as frolinic acid; aceglatone; aldophosphamide glycoside; aminolevulinic acid; eniluracil; amsacrine; bestrabucil; bisanrrene; edatraxate; defofamine; demecolcine; diaziquone; elfornithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; podophyllinic acid; 2- ethylhydrazide; procarbazine; PSK^{®} polysaccharide complex (JHS Natural Products, Eugene, OR); razoxane; rhizoxin; sizofiran; spirogermanium; tenuazonic acid; triaziquone; 2,2^2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; taxoids, e.g., TAXOL^{®} paclitaxel (Bristol- Myers Squibb Oncology, Princeton, N.J.), ABRAXANE^{™} Cremophor-free, albumin-engineered nanoparticle formulation of paclitaxel (American Pharmaceutical Partners, Schaumberg, Illinois), and TAXOTERE^{®} doxetaxel (Rhone- Poulenc Rorer, Antony, France); chloranbucil; GEMZAR^{®} gemcitabine; 6-thioguanine; mercaptopurine; methotrexate; platinum analogs such as cisplatin and carboplatin; vinblastine; platinum; etoposide (VP-16); ifosfamide; mitoxantrone; vincristine; NAVELBINE^{®} vinorelbine; novantrone; teniposide; edatrexate; daunomycin; aminopterin; xeioda; ibandronate; CPT-11; topoisomerase inhibitor RFS 2000; difluorometlhylornithine (DMFO); retinoids such as retinoic acid; capecitabine; and pharmaceutically acceptable salts, acids or derivatives of any of the above.

Also included are: (i) anti-hormonal agents that act to regulate or inhibit hormone action on tumors such as anti-estrogens and selective estrogen receptor modulators (SERMs), including, for example, tamoxifen (including NOLVADEX^{®}; tamoxifen citrate), raloxifene, droloxifene, 4-hydroxytamoxifen, trioxifene, keoxifene, LY 1 17018, onapristone, and FARESTON^{®} (toremifine citrate); (ii) aromatase inhibitors that inhibit the enzyme aromatase, which regulates estrogen production in the adrenal glands, such as, for example, 4(5)-imidazoles, aminoglutethimide, MEGASE^{®} (megestrol acetate), AROMASIN^{®} (exemestane; Pfizer), formestanie, fadrozole, RI VISor^{®} (vorozole), FEMARA^{®} (letrozole; Novartis), and ARIMIDEX^{®} (anastrozole; AstraZeneca); (iii) antiandrogens such as flutamide, nilutamide, bicalutamide, leuprolide, and goserelin; as well as troxacitabine (a 1 ,3-dioxolane nucleoside cytosine analog); (iv) protein kinase inhibitors such as ME inhibitors (WO 2007/044515); (v) lipid kinase inhibitors; (vi) antisense oligonucleotides, particularly those which inhibit expression of genes in signaling pathways implicated in aberrant cell proliferation, for example, PKC-alpha, Raf and H-Ras, such as oblimersen (GENASENSE^{®}, Genta Inc.); (vii) ribozymes such as VEGF expression inhibitors (e.g., ANGIOZYME^{®}) and HER2 expression inhibitors; (viii) vaccines such as gene therapy vaccines, for example, ALLOVECTIN^{®}, LEUVECTIN^{®}, and VAX1 D^{®}; PROLEUKIN^{®} rIL-2; topoisomerase 1 inhibitors such as LURTOTECAN^{®}; ABARELIX^{®} rmRH; (ix) anti-angiogenic agents such as bevacizumab (AVASTIN^{®}, Genentech); and pharmaceutically acceptable salts, acids and derivatives of any of the above.

Protein kinase inhibitors include tyrosine kinase inhibitors which inhibit to some extent tyrosine kinase activity of a tyrosine kinase such as an ErbB receptor. Examples of tyrosine kinase inhibitors include EGFR- 10 targeted drugs such as: (i) antibodies which bind to EGFR, including MAb 579 (ATCC CRL HB 8506), MAb 455 (ATCC CRL HB8507), MAb 225 (ATCC CRL 8508), MAb 528 (ATCC CRL 8509) (see, US 4943533, Mendelsohn et al.) and variants thereof, such as chimerized 225 (C225 or Cetuximab; ERBITUX^{®}, Imclone) and reshaped human 225 (H225) (WO 96/40210, Imclone Systems Inc.); antibodies that bind type Π mutant EGFR (US 5212290); humanized and chimeric antibodies that bind EGFR (US 5891996); and human antibodies that bind EGFR, such as ABX-EGF (WO 98/50433); (ii) anti-EGFR antibody conjugated with a cyotoxic agent (EP 659439A2); and small molecules that bind to EGFR including ZD1839 or Gefitinib QRESSA^{™}; Astra Zeneca), Erlotinib HC1 (CP-358774, TARCEVA^{™}; Genentech/OSI) and AG1478, AG1571 (SU 5271; Sugen), quinazolines such as PD 153035,4-(3-chloroanilino) quinazoline, pyridopyrimidines, pyrirnidopyrirnidines, pyrrolopyrimidines, such as CGP 59326, CGP 60261 and CGP 62706, and pyrazolopyrimidines, 4- 20 (phenylamino)-7H-pyrrolo[2,3-dJ pyrimidines, curcumin (diferuloyl methane, 4,5-bis(4- fluoroanilino)phthaiimide), tyrphostines containing nitrothiophene moieties; PD-0183805 (Warner-Lambert) and antisense molecules (e.g., those that bind to ErbB-encoding nucleic acid).

In yet another embodiment, the ADC cytotoxic agent of the present invention is an anti-tubulin agent. In more specific embodiments, the cytotoxic agent is selected from the group consisting of vinca alkaloids, podophyllotoxins, taxanes, baccatin derivatives, cryptophycin, maytansinoids, combretastatins, and dolastatin. In a more specific embodiment, the cytotoxic agent is vincristine, vinblastine, vindesine, vinorelbine, VP-16, camptothecin, paclitaxel, docetaxel, episilon A, episilon B, nocodazole, colchicine, cortisimide, estramustine, semadotine, discodermolide, maytansine DM-1, auristatin or other dolastatin derivatives, such as auristatin E or auristatin F, AEB, AEVB, AEFP, MMAE (monomethyl auristatin E), MMAF (monomethyl auristatin F), eruterobin or netropsin.

In addition, powerful chemotherapeutic agents such as CC-1065 analogs, calikiamycin, maytansine, dolastatin 10 analogs, lysoxin, and palytoxin can be linked to ADCs using conditionally stable linkers to form potent immunoconjugates.

In other embodiments, an antibody of the invention or fragment or variant thereof is conjugated to a therapeutic agent or drug moiety that modifies a given biological response. The therapeutic agent or drug moiety is not limited to classic chemotherapeutic agents. For example, the drug moiety may be a protein or polypeptide that possesses the desired biological activity. Such proteins include, for example, toxins such as abrin, ricin A, Pseudomonas exotoxin, cholera toxin, or diphtheria toxin; proteins such as tumor necrosis factor, α-interferon, β-interferon, nerve growth factor, platelet-derived growth factor Tissue plasminogen activator, apoptotic agents such as TNF-α, TNF-β, AIMI, AIMII, Fas ligand, and VEGf, thrombotic or antiangiogenic agents such as angiostatin or endostatin; or biological response modifiers For example, lymphokines (e.g., interleukin-1 (IL-1), interleukin-2 (IL-2), interleukin-4 (IL-4), interleukin Kin-6 (IL-6), Interleukin-7 (IL-7), Interleukin-9 (IL-9), Interleukin-15 (IL-15), Interleukin-12 (IL-12), Granules Sphere macrophage colony stimulating factor (GM-CSF), and granulocyte colony stimulating factor (G-CSF)), or growth factors (e.g., growth hormone (GH)).

In another embodiment the cytotoxic agent is an agent for immunotherapy.

Cancer immunotherapy attempts to stimulate the immune system to reject and destroy tumors. Tumors have been found to be able to escape host immunity by manipulating the tumor microenvironment and driving immunosuppression, such that although the components necessary for mounting an effective anti-tumor immune response are present in patients with cancer, the host usually fails to arrest tumor progression (8). Numerous molecular and cellular mechanisms have been proposed in the past to explain this counterintuitive scenario, largely focusing on the "immune escape" of tumors and suggesting that tumors develop the capability to avoid tumor-specific immune responses generated by the host, or they altogether disable the host anti-tumor immunity. This process, referred to as "tumor-induced immune suppression" has been recognized in recent years and is being intensively investigated. It appears that tumors can interfere with all components of the immune system, affecting all stages of the anti-tumor immune response.

Molecular alterations that occur in tumor cells as the tumor progresses from the premalignant to metastatic phenotype are a result of genetic instability, now recognized as a principal characteristic of all tumors. Genetic changes are detectable during early stages of tumorigenesis, become more pronounced as the tumor progresses, are the greatest in metastatic cells and are responsible for tumor heterogeneity and alterations in the antigenic epitope profile of tumor cells. It has been suggested that the immune system might drive these antigenic changes via "immune editing" by eliminating those malignant cells that are sensitive to immune intervention and allowing for the selection and survival of immune resistant variants. The net result of immune editing is that the tumor escapes from the host immune system. Regulatory T cells (Treg) and myeloid-derived suppressor cells (MDSC) are two types of cells used by the tumor to execute an immune escape. Protection of immune cells from the adverse effects of Treg, MDSC or inhibitory factors, and thus enhancement of their effector functions, can restore effective anti-tumor immunity in patients with cancer.

CD4CD25highFOXP3* Treg cells accumulate in human tumors and in the peripheral circulation of patients with cancer. It is unclear whether these cells migrate to tumors or expand in situ. Because tumor-associated antigens (TAA) are self-antigens, it is possible that Treg accumulation is a response to enforce immune tolerance. Treg cells contribute to the down-regulation of immune activity of effector T cells by a variety of mechanisms including IL-10 and TGF- 1 production, enzymatic degradation of ATP to immunosuppressive adenosine, or the engagement of the Fas/FasL and granzyme/perforin pathways. Tumors benefit from immunosuppressive effects mediated by Treg.

Bone marrow-derived immature myeloid cells MDSC (CD34*CD33CD13+CD15-) are present at an elevated frequency in the peripheral circulation and tumors of nearly all cancer patients. They are recruited by tumor-derived soluble factors such as TGF-31, IL-10, VEGF, GMCSF, IL-6, PGE2. They promote tumor growth by suppressing T-cell responses via several mechanisms, including production of arginase-1, an enzyme involved in L-arginine metabolism, as well as activation of inducible nitric oxide synthase (iNOS). They also control the tumor's production of indoleamine-2, 2 dioxygenase (IDO), which is involved in the catabolism of tryptophan, an amino acid essential for T-cell differentiation.

Bacillus Calmette-Guerin (BCG) immunotherapy for early stage (non-invasive) bladder cancer utilizes instillation of attenuated live bacteria into the bladder, and is effective in preventing recurrence in up to two thirds of cases. The immune response to BCG can be summarized as follows (9): infection of urothelial and bladder tumor cells by BCG results in internalization of BCG, which increases the expression of antigen-presenting molecules. This induces an immune response via the release of cytokines, such as Th1 cytokines (IL-2, tumor necrosis factor, IL-12, and IFN-y) and Th2 cytokines (IL-4, IL-5, IL 6, and IL-10) along with IL-8 and IL-17. This complex immune cascade induces antitumor activity mediated by cytotoxic T lymphocytes, natural killer cells, neutrophils, and macrophages. In another study of BCG in an orthotopic mouse bladder tumor model, the population of MDSCs was significantly downregulated following the high-dose BCG therapy compared with the low-dose therapy. In the same study, CD4*/Foxp3' Tregs also exhibited the same change. Following the BCG treatment, the population of CD4*/Foxp3' Tregs was decreased in the blood. These inhibitory effects on the immune-suppressive factors may explain the robust antitumor therapeutic effects of the BCG therapy (10).

Immune checkpoint regulators, which can be both costimulatory and coinhibitory molecules, regulate the immune system. The balance between these checkpoints signals regulates lymphocyte activation and consequently the immune response. Tumors can use these checkpoint regulators to protect themselves from the immune system. Immune checkpoint therapies can enhance the proliferation, migration, persistence, and/or cytotoxic activity of T cells in a subject and, in particular, by increasing the numbers of tumor infiltrating T cells (11). The best characterized immune checkpoint receptors are cytotoxic T-lymphocyte-associated antigen 4 (CTLA-4; also known as CD152), programmed cell death protein 1 (PD-1; also known as CD279), and indoleamine 2,3-dioxygenase (IDO), and agents targeting these molecules are either approved or being extensively tested in clinical trials for treatment of multiple solid or hematological cancers.

CTLA-4, an inhibitory receptor, is a global immune checkpoint regulator engaged in priming immune responses via down-regulating the initial stages of T-cell activation. CTLA-4 was the first clinically validated checkpoint pathway target. CTLA-4 is homologous to the T-cell costimulatory protein CD28, and both molecules bind to CD80 and CD86 on antigen-presenting cells. CTLA-4 binds CD80 and CD86 with a markedly higher affinity and avidity than CD28 does, enabling CTLA-4 to outcompete CD28 for its ligands, resulting in an effective inhibition of T cell activation (12). Blocking CTLA-4 activity by using an antagonistic antibody interferes with this mechanism and thus preserves the activity of the T cells. Currently, Bristol-Myers Squibb's anti-CTLA-4 mAb, ipilimumab, has received US Food and Drug Administration (FDA) approval for patients with metastatic melanoma. Additionally, AstraZeneca's anti-CTLA-4 mAb, tremelimumab, has been granted orphan drug designation by the FDA for the treatment of patients with malignant mesothelioma.

PD-i is another inhibitory receptor expressed on activated T and B cells, and it functions to dampen the immune response (13). PD-i acts as an immune checkpoint regulator, and upon binding of one of its ligands, PD-Li (B7-H1, CD274) or PD-L2 (B7 DC, CD273), PD-i inhibits proliferation and cytokine production of T cells. Overexpression of PD-Li or PD-L2 in the tumor microenvironment leads to the inhibition of the intratumoral immune responses (14). Inhibition of the interaction between PD-i and PD-L1 by anti-PD-i/PD-Li antibodies can inhibit the deactivation of T cells, and thus enhance anti-tumor responses, delay tumor growth, and facilitate tumor rejection (15). Two anti-PD I mAbs, Bristol-Myers Squibb's nivolumab, and Merck's pembrolizumab, have received US FDA approval for patients with metastatic melanoma and non-small-cell lung cancer. Recently, the FDA approved nivolumab as a treatment for patients with metastatic renal cell carcinoma. In 2016, Roche's anti-PD-Li mAb, atezolizumab, was approved by the FDA for treating metastatic urothelial cancer and non-small cell lung cancer. Other anti-PD-LI mAbs, such as AstraZeneca's durvalumab and Pfizer's avelumab, are in late-stage clinical trials.

Indoleamine 2,3-dioxygenase (IDO) is an enzyme that catalyzes the oxidative cleavage of tryptophan (16). IDO plays a role in suppressing the immune system because T cells undergoing antigen-dependent activation require tryptophan for cell proliferation and survival (17). IDO is overexpressed in most tumors and/or tumor-draining lymph nodes and it plays a significant role in helping tumors to evade attack from the immune system. IDO inhibitors block the IDO enzyme which reduces the depletion of tryptophan and ultimately may help to promote an enhanced immune response against the tumor (18). Currently, a number of clinical trials are underway to evaluate IDO inhibitors for both monotherapy and combination cancer therapies.

In addition to PD-1, CTLA-4, and IDO, other immune checkpoints are also involved in the occurrence and development of malignant tumors, including T cell membrane protein-3 (TIM-3), LAG3, T-cell immunoreceptor with Ig and immunoreceptor tyrosine-based inhibitory motif (ITIM) domains (TIGIT), BTLA, inducible T-cell costimulator (ICOS), killer inhibitory receptors (KIR), and V-domain Ig-containing suppressor of T cell activation (VISTA). Similar to PD-1, CTLA-4 and IDO, these immune checkpoints inhibit lymphocyte activity and/or induce lymphocyte anergy, and thus are ideal targets for cancer immunotherapy. Blocking antibodies for these immune checkpoints have shown specific anti-tumor activities in animal models, and some are being tested in clinical trials.

Thus, the cytotoxic agent can also be any immunotherapy agent that enhances the anti cancer effect of the antibodies anti-IL11RA, or an analogue or pharmaceutically acceptable salt thereof. In some embodiments, the cytotoxic agent is an immunologic agent that can stimulate an effective immune response and/or inhibit immune-suppression. In some embodiments, the cytotoxic agent is an agent that modulates, particularly inhibits and downregulates immune-suppressive factors such as regulatory T cells and MDSCs, including any inhibitors or antibodies of the programmed cell death 1 ligand 1 (PD-LI)/ programmed cell death protein 1 (PD-i) pathway. In some embodiments, the cytotoxic agent are any effective inhibitors/antibodies capable of modulating immunocytes activities including but not limited to targeting cytotoxic T lymphocyte-associated antigen 4 (CTLA-4), CD20, CD19, IL-i7a, CD25, arginase I (ARG1), indoleamine-2,3-dioxygenase (IDO), or tryptophan 2,3 dioxygenase (TDO2). In some embodiments, the second inhibitor is BCG. In particular embodiments, the cytotoxic agent is an antibody against PD-i or an antigen binding fragment thereof. In other particular embodiments, the cytotoxic agent is an antibody against PD-Li or an antigen binding fragment thereof. In other particular embodiments, the cytotoxic agent is an antibody against CTLA-4 or an antigen binding fragment thereof. In other particular embodiments, the cytotoxic agent is a BCG therapy, preferably an attenuated BCG therapy, more preferably mycobacterial cell wall fragments, and most preferably mycobacterial cell wall fragments with biologically active nucleic acids derived from Mycobacterium phlei.

Techniques for conjugating therapeutic moieties to antibodies are well known. The therapeutic moiety may be conjugated to the antibody by any method known in the art including, but not limited to, aldehyde / Schiff bond, sulfhydryl bond, acid labile bond, cis-aconityl (Aconityl) bond, hydrazone bond, and enzyme-degradable bond. The fusion with the therapeutic portion of the antibody need not be direct, but may occur through a linker sequence. Such linker molecules are generally known in the art.

In some embodiments, the conjugation of the drug to the antibody is performed by a linker that is sensitive to the environment (e.g., the lysosomal environment due to the endosomal environment or pH or protease sensitivity).

In one embodiment, the linker is an acid labile hydrazone or hydrazide group that is hydrolyzed within the lysosome. In other embodiments, the drug can be conjugated to the antibody via other acid labile linkers such as cis-aconitic amides, orthoesters, acetals and ketals. Such linkers are relatively stable under neutral pH conditions, such as blood conditions, but are unstable at pH 5 or lower, i.e., at a pH close to lysosomes.

In other embodiments, the drug binds to the antibody of the invention using a peptide spacer that is cleaved by intracellular proteases. Target enzymes include cathepsins B and D and plasmin, all of which are known to hydrolyze dipeptide drug derivatives resulting in the release of active drugs inside the target cell

In certain non-limiting embodiments of the present invention, the linker region between the drug and antibody portions of the ADC can be cleaved under certain conditions, with linker cleavage or hydrolysis. The drug moiety is released from the antibody moiety. Preferably, this linker is sensitive to cleavage or hydrolysis under intracellular conditions.

In one embodiment, the linker region between the drug and antibody portions of the ADC can be hydrolyzed if the pH changes by a certain value or exceeds a certain value.

In yet another specific embodiment, the linker is a disulfide linker. Various disulfide linkers are known in the art, including but not limited to SATA (N-succinimidyl-S-acetylthioacetate), SPDP (N-succinimidyl-3- (2-pyridyldithio) propionate), SPDB (N-succinimidyl-3-(2-pyridyldithio) butyrate) and SMPT (N-succinimidyl-oxycarbonyl-α-methyl-α-(2-pyridyldithio)toluene) Is included. SPDB and SMPT.

### Methods for Producing Antibodies

Antibodies or fragments thereof can be produced by any method known in the art for antibody synthesis, particularly chemical synthesis, or preferably by recombinant expression techniques.

### Method of detection

In a sixth aspect, the invention relates to a method for detecting the IL-11 receptor in a sample which comprises contacting the sample with the antibody of the invention and detecting in said sample the formation of a complex between the antibody and the IL-11 receptor.

As used herein the term "detecting" and derivations of this term refer to determining the presence, absence, amount, localization, distribution or organization of an analyte in a sample. In the present case, the analyte is the IL-11 receptor and the sample is a cell population or an organism. According to the present invention, "detecting" also includes visualizing, determining, observing, evaluating, quantifying, measuring or imaging the IL-11 receptor.

The sample may be any sample that is suspected of containing the IL-11 receptor, such as, for example, a tissue section or specimen, a homogenized tissue extract, a cell, an organelle, separated and/or purified forms of any of the above antigen-containing compositions, or any biological fluid, including blood, serum and plasma. Preferably, the sample suspected of containing the IL-11 receptor a biopsy tissue or cell population, blood, serum or plasma.

The term "contacting", as used herein refers to the process by which the antibody of the invention comes into contact with the cell population or the organism and includes any possible method that allows the introduction of the antibody into the cell population or the organism and therefore, to specifically bind to the IL-11 receptor.

Contacting the chosen biological sample with the antibody of the invention under effective conditions and for a period of time sufficient to allow the formation of immune complexes is generally a matter of simply adding the antibody composition to the sample and incubating the mixture for a period of time long enough for the antibodies to form immune complexes. Usually, the contacting process is carried out under conditions adequate for the formation of the complex.

"Under conditions adequate for the formation of a complex" means that the conditions preferably include diluting the antigens and/or antibodies with solutions such as BSA, bovine gamma globulin (BGG) or phosphate buffered saline (PBS)/Tween. These added agents also tend to assist in the reduction of nonspecific background.

The "suitable" or "adequate" conditions also mean that the incubation is at a temperature or for a period of time sufficient to allow effective binding. Incubation steps are typically from about 1 to 2 to 4 hours or so, at temperatures preferably on the order of 25° C. to 27° C., or may be overnight at about 4° C. or so.

The determination of the amount of complex formed may be done in a number of ways. In a preferred embodiment, the antibody is labelled, and binding determined directly. For example, this may be done by attaching the IL-11 receptor to a solid support, adding the labelled antibody (for example a fluorescent label), washing off excess reagent, and determining whether the label is present on the solid support. Various blocking and washing steps may be utilized as is known in the art.

In general, the detection of immunocomplex formation is well known in the art and may be achieved through the application of numerous approaches. These methods are generally based upon the detection of a label or marker, such as any of those radioactive, fluorescent, biological and enzymatic tags. U.S. Patents concerning the use of such labels include U.S. Pat. Nos. 3,817,837; 3,850,752; 3,939,350; 3,996,345; 4,277,437; 4,275,149 and 4,366,241. Of course, one may find additional advantages through the use of a secondary binding ligand such as a second antibody and/or a biotin/avidin ligand binding arrangement, as is known in the art.

In the present case, the determination of the complex of the method of the invention is carried out using antibody of the invention.

In another embodiment, the antibody is immobilized.

As the person skilled in the art will understand that there is a wide range of conventional assays that can be used in the present invention which use an antibody of the invention that is not labelled (primary antibody) and an antibody of the invention that is labelled (secondary antibody); these techniques include Western blot or immunoblot, ELISA (Enzyme-Linked Immunosorbent Assay), RIA (Radioimmunoassay), competitive EIA (Competitive Enzyme Immunoassay), DAS-ELISA (Double Antibody Sandwich-ELISA), immunocytochemical and immunohistochemical techniques, flow cytometry or multiplex detection techniques based on using protein microspheres, biochips or microarrays which include the antibody of the invention. Other ways of detecting and quantifying the IL-11 receptor using the antibody of the invention include affinity chromatography techniques or ligand binding assays.

It will also be understood that antibodies that are not labelled need to be detected with an additional reagent, for example, a secondary antibody that is labelled, which will be labelled. This is particularly useful in order to increase the sensibility of the detection method, since it allows the signal to be amplified.

In addition, the detection of the antibody can also be carried out by detecting changes in the physical properties in the sample that occur as a result of the binding of the antibody to its cognate antigen. These assays include determining a transmission-related parameter in a sample, which are known in the art. The term "transmission-related parameter", as used herein, relates to a parameter indicating or correlating with the ratio of transmitted light versus incident light of a sample or to a parameter derived therefrom.

In an embodiment, a transmission-related parameter is determined by turbidimetry or by nephelometry.

Turbidimetry, as used herein, refers to the measurement of light-scattering species in solution by means of a decrease in intensity of the incident beam after it has passed through solution. For turbidimetric assays, the change in the amount of light absorbed (inverse of amount transmitted) can be related to the amount of agglutination which occurs. Hence, the amount of analyte (the species causing agglutination) in the sample can be easily determined.

Nephelometry, as used herein, refers to a technique for measuring the light-scattering species in solution by means of the light intensity at an angle away from the incident light passing through the sample. Nephelometric assays present an indirect method of measurement of the amount of analyte in a sample by measuring the amount of light scattered or reflected at a given angle (typically 90) from the origin. In the presence of the protein antigen, the antibody reacts with the antigen, and a precipitation reaction begins. The measurement is taken early in this precipitation reaction time sequence. A quantitative value is obtained by comparison with a standard curve, which has been established previously. In order to increase the sensitivity of the detection, the antibody can be adsorbed or covalently attached to polymeric microspheres. In this way, a greater signal is produced with less reagent.

The detection method based on turbidimetry or nephelometry according to the present disclosure works with all known agglutination tests with and without microparticles enhancement. Typically used within the present disclosure is a "microparticle-enhanced light scattering agglutination tests" which is also called "particle-enhanced turbidimetric immunoassays" (PETIA). Agglutination-based immunoassays are routinely used in clinical diagnostics for the quantitation of serum proteins, therapeutic drugs and drugs of abuse on clinical chemistry analyzers, because they have the benefits of being quasi-homogeneous assays which do not require any separation or wash step. To enhance the optical detection between the antigen to be detected and the specific antibody in the reaction mixture, the antibody may be linked to suitable particles. Thereby, the antigen reacts and agglutinates with the particles which are coated with antibody. With increasing amount of antibody, the agglutination and the size of the complexes are increasing, leading further to a change of light scattering.

In another embodiment, the binding of the antibody to its cognate antigen can be detected by Surface plasmon resonance (SPR).

As used herein, SPR refers to a phenomenon that the intensity of a reflected light decreases sharply at a particular angle of incidence (i.e., an angle of resonance) when a laser beam is irradiated to a metal thin film. SPR is a measurement method based on the phenomenon described above and is capable of assaying a substance adsorbed on the surface of the metal thin film, which is a sensor, with high sensitivity. According to the present invention, for example, the target substance in the sample can then be detected by immobilizing one or more antibodies according to the present invention on the surface of the metal thin film beforehand, allowing the sample to pass through the surface of the metal thin film, and detecting the difference of the amount of the substance adsorbed on the surface of the metal thin film resulting from the binding of the antibody and the target antigen, between before and after the sample passes therethrough.

### Pharmaceutical composition and medical uses using the antibody of the invention

In a seventh aspect, the invention relates to pharmaceutical composition comprising the antibody of the invention or the ADC according to claim 9 and at least one pharmaceutically acceptable excipient and/or vehicle.

The term "pharmaceutical composition" is such a form that allows the biological activity of the active ingredient contained therein to be effective and has unacceptable toxicity for the subject to which the composition is administered. Refers to a preparation that does not contain additional ingredients.

"Pharmaceutically acceptable carrier" refers to an ingredient of a pharmaceutical composition other than an active ingredient that is non-toxic to a subject. Pharmaceutically acceptable carriers include but are not limited to buffers, excipients, stabilizers or preservatives.

Pharmaceutical compositions and formulations as described herein can be prepared by mixing the active ingredients having the desired degree of purity with one or more optional pharmaceutically acceptable carriers (Remington's Pharmaceutical Sciences 22nd edition, 2012), in the form of lyophilized formulations or aqueous solutions. Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counterions such as sodium; metal complexes (e.g. Zn- protein complexes); and/or non-ionic surfactants such as polyethylene glycol (PEG). Exemplary pharmaceutically acceptable carriers herein further include interstitial drug dispersion agents such as soluble neutral-active hyaluronidase glycoproteins (sHASEGP), for example, human soluble PH-20 hyaluronidase glycoproteins, such as rHuPH20 (HYLENEX^{®}, Baxter International, Inc.).

The present invention provides methods for immunotherapy comprising administering a therapeutic effective amount of the antibody of the present invention.

Thus, in an eighth aspect, the invention relates to the antibody or the ADC of the invention for use in medicine.

In addition, it is been reported that neutralizing antibodies that block IL11 signaling reduce fibrosis, steatosis, hepatocyte death, inflammation and hyperglycemia in mice with diet-induced steatohepatitis (Widjaja AA et al. Gastroenterology. 2019;157:777-792.e14. doi:10.1053/j.gastro.2019.05.002) and that the up-regulation of IL11 in the lung of patients with idiopathic pulmonary fibrosis (IPF) is associated with disease severity (Ng B, et al. Sci Transl Med. 2019;11 (511):eaaw1237. doi:10.1126/scitranslmed.aaw1237).

Thus, in a ninth aspect, the invention relates to the antibody or the ADC of the invention for use in a method of preventing or treating a disease characterized by increased IL11RA signaling, wherein the disease is selected from pulmonary fibrosis, liver fibrosis, liver inflammation, steatosis, steatohepatitis, NASH and cancer.

The expression "increased IL11RA signaling" or "increased IL11RA activity" in a test subject or a biological sample refers to higher total IL11RA activity in the test subject or biological sample in comparison with a control, e.g., a healthy subject or a standard sample. Preferably, although not necessarily, the activity is at least 10%, more preferably at least 50%, even more preferably at least 100%, and still more preferably at least 150% higher in the test subject or sample than in the control. The increased activity, for example, may result from increased basal IL11RA activity, prolonged stimulation, delayed degradation, or over-expression, e.g., due to enhanced ligand binding, promiscuous or inappropriate ligand binding, constitutive receptor activation, impaired recycling resulting in augmentation of signaling or delayed degradation.

The term "control" or "reference value", as used herein, relates to a predetermined criteria used as a reference for evaluating the values or data obtained from the samples collected from a subject. The reference value or reference level can be an absolute value; a relative value; a value that has an upper or a lower limit; a range of values; an average value; a median value; a mean value; or a value as compared to a particular control or baseline value. A reference value can be based on an individual sample value, such as for example, a value obtained from a sample from the subject being tested, but at an earlier point in time. The reference value can be based on a large number of samples, such as from population of subjects of the chronological age matched group, or based on a pool of samples including or excluding the sample to be tested. In one embodiment, the reference value corresponds to the levels IL11RA signaling levels determined in a healthy subject, whereby a healthy subject is understood as a subject that shows no increased IL11RA signaling at the moment the levels IL11RA activation is determined.

In another embodiment, the reference value corresponds to an average or mean level of the corresponding IL!! RA signaling from a pool of samples obtained from a group of patients who are well documented from the clinical point of view, and who present no disease, particularly who are not suffering from ischemic tissue damage, particularly not suffering from pulmonary fibrosis, liver fibrosis, liver inflammation, steatosis, steatohepatitis, NASH or cancer. In said samples, the IL11RA activation levels can be determined, for example by verification of STAT1 and STAT3 activation level upon stimulation with IL11, as shown in the examples of the present application. In the determination of the reference value, it is necessary to take into consideration some characteristics of the type of sample, such as age, gender, the physical state or other characteristics of the patient. For example, the reference sample can be obtained from identical amounts of a group of at least 2, at least 10, at least 100 to more than 1000 individuals, such that the population is statistically significant..

As used herein, the terms "treat", "treatment", or "amelioration" refers to therapeutic treatment, the purpose of which is to reverse, reduce, suppress, delay or stop the progression or severity of the condition associated with the disease or disorder. The term "treatment" includes reducing or alleviating at least one adverse effect or condition of a condition, such as cancer, a disease or disorder. Treatment is usually "effective" when one or more symptoms or clinical markers are reduced. Alternatively, treatment is "effective" if disease progression is delayed or halted. That is, "treatment" includes not only the improvement of symptoms or markers, but also the interruption of at least a condition that indicates the progression or worsening of symptoms that would be expected in the absence of treatment. The beneficial or desirable clinical outcome, whether detectable or not, is a reduction in one or more symptoms, a reduction in the extent of the disease, a stable (i.e., not aggravated) condition of the disease, a disease These include, but are not limited to, delayed or slowed progression, amelioration or alleviation of the disease state, and remission (partial or total). The term "treatment" of a disease also includes providing relief from symptoms or side effects of the disease (including symptomatic treatment).

The term "pulmonary fibrosis" refers to a condition in which the lungs become scarred over time. Pulmonary fibrosis may be a secondary effect of other diseases. Most of these are classified as interstitial lung diseases. Examples include autoimmune disorders, viral infections and bacterial infection like tuberculosis which may cause fibrotic changes in both lung's upper or lower lobes and other microscopic injuries to the lung. However, pulmonary fibrosis can also appear without any known cause. In this case, it is termed "idiopathic". In this regard, it is been reported that the upregulation of in the lung of patients with IPF, associated with disease severity (Ng B, Dong J, D'Agostino G, et al. Interleukin-11 is a therapeutic target in idiopathic pulmonary fibrosis. Sci Transl Med. 2019;11(511):eaaw1237. doi:10.1126/scitranslmed.aaw1237).

Diseases and conditions that may cause pulmonary fibrosis as a secondary effect include
- Inhalation of environmental and occupational pollutants, such as metals in asbestosis, silicosis and exposure to certain gases. Coal miners, ship workers and sand blasters among others are at higher risk.
- Hypersensitivity pneumonitis, most often resulting from inhaling dust contaminated with bacterial, fungal, or animal products.
- Cigarette smoking can increase the risk or make the illness worse.
- Some typical connective tissue diseases such as rheumatoid arthritis, ankylosing spondylitis SLE and scleroderma
- Other diseases that involve connective tissue, such as sarcoidosis and granulomatosis with polyangiitis.
- Infections
- Certain medications, e.g. amiodarone, bleomycin (pingyangmycin), busulfan, methotrexate, apomorphine, and nitrofurantoin
- Radiation therapy to the chest

In the present invention, pulmonary fibrosis includes not only pulmonary fibrosis in a restricted sense, but also pulmonary fibrosis in a broad sense that includes co-existence of interstitial pneumonia. The pulmonary fibrosis of the present invention can be caused by any interstitial pneumonia, for example, infectious interstitial pneumonia associated with viral pneumonia, fungal pneumonia, mycoplasmal pneumonia, etc., interstitial pneumonia associated with collagen disease such as rheumatoid arthritis, systemic scleroderma, dermatomyositis, polymyositis, mixed connective-tissues disease (MCTD), etc., interstitial pneumonia associated with radiation exposure, drug-induced interstitial pneumonia caused by anticancer agents such as bleomycin, herbal medicines such as Sho-sai-ko-to, interferon, antibiotics, paraquat, etc., and idiopathic interstitial pneumonia such as idiopathic pulmonary fibrosis, nonspecific interstitial pneumonia, acute interstitial pneumonia, cryptogenic organizing pneumonia, respiratory bronchiolitis-associated interstitial lung disease, desquamative interstitial pneumonia, lymphoid interstitial pneumonia, etc., and accordingly, it also includes chronic states of such interstitial pneumonia. The pulmonary fibrosis of the present invention preferably includes chronic states of drug-induced interstitial pneumonia and idiopathic interstitial pneumonia.

The term "liver fibrosis" refers to the formation of an abnormally large amount of scar tissue in the liver. Fibrosis develops when the liver is repeatedly or continuously damaged. After a single episode of injury, even if severe (as with acute hepatitis), the liver commonly repairs itself by making new liver cells and attaching them to the web of connective tissue (internal structure) that is left when liver cells die. However, if injury is repeated or continuous (as occurs in chronic hepatitis), liver cells attempt to repair the damage, but the attempts result in scar tissue (fibrosis). Fibrosis can develop more rapidly when it is caused by a blockage in the bile ducts.

Scar tissue replaces the liver cells and, unlike liver cells, performs no function. Scar tissue can distort the liver's internal structure and interfere with blood flow to and in the liver, limiting the blood supply for the liver cells. Without enough blood, these cells die, and more scar tissue is formed. Also, blood pressure in the vein that carries blood from the intestine to the liver (portal vein) increases-a condition called portal hypertension.

The term "steatosis" as used herein, is meant to include all stages of fatty liver development from the initial asymptomatic phase with small amounts of accumulated fat in the liver to the later stages of more severe fatty liver. More severe later stages of the disorder are referred to as steato-hepatitis, which may be accompanied by a progressive inflammation of the liver, hepatocyte necrosis and/or cirrhosis, characterized by replacement of liver tissue by fibrosis, scar tissue and regenerative nodules (lumps that occur as a result of a process in which damaged tissue is regenerated), which gradually and ultimately leads to loss of liver function.

Steatosis can arise from several different causes. One of the most prominent causes of steatosis is excessive alcohol intake. steatosis arising as a result of excessive alcohol intake, usually more than 20-30 g alcohol or more than 2 standard drinks per day, is also referred to as alcohol-dependent steatosis or steato-hepatitis or alcoholic steatosis or steato-hepatitis. Other causes of steatosis are collectively called non-alcoholic steato-hepatitis (NASH). One major cause of steatosis is obesity (with or without effects of insulin resistance). Obese individuals are more prone to steatosis. However, the condition is also associated with other diseases that influence fat metabolism. Alcoholic steatosis and non-alcoholic steatosis are not easily distinguished morphologically, and both show micro-vesicular and macrovesicular fatty changes at different stages.

Another major cause of steatosis or steato-hepatitis is viral infection. Viral hepatitis may exist in acute (recent infection, relatively rapid onset) or chronic forms. The most common causes of viral hepatitis are the five unrelated hepatotropic viruses Hepatitis A, Hepatitis B, Hepatitis C, Hepatitis D, and Hepatitis E. In addition to the hepatitis viruses, other viruses that can also cause hepatitis include Herpes simplex, Cytomegalovirus, Epstein-Barr virus, and Yellow fever.

As used herein, the term "Nonalcoholic steatohepatitis" or "NASH" refers to liver inflammation and damage caused by a buildup of fat in the liver. NASH is part of a group of conditions called nonalcoholic fatty liver disease (NAFLD). NASH resembles alcoholic liver disease, but occurs in people who drink little or no alcohol. The major feature in NASH is fat in the liver, along with inflammation and damage. Most people with NASH feel well and are not aware that they have a liver problem. Nevertheless, NASH can be severe and can lead to cirrhosis, in which the liver is permanently damaged and scarred and no longer able to work properly. NASH is usually first suspected in a person who is found to have elevations in liver tests that are included in routine blood test panels, such as alanine aminotransferase (ALT) or aspartate aminotransferase (AST). When further evaluation shows no apparent reason for liver disease (such as medications, viral hepatitis, or excessive use of alcohol) and when x rays or imaging studies of the liver show fat, NASH is suspected. The only means of proving a diagnosis of NASH and separating it from simple fatty liver is a liver biopsy.

In a particular embodiment, the disease characterized by increased IL11RA signaling is cancer. Thus, in some embodiments, treating cancer includes reducing tumor volume, reducing the number of cancer cells, suppressing cancer metastasis, prolonging life, reducing cancer cell growth, reducing cell survival, or reducing cancerous status It involves amelioration of the various physiological symptoms involved. In a particular embodiment, the cancer is lung cancer, colon cancer, breast cancer, liver cancer, endometrial cancer, stomach cancer and prostate cancer. In a more preferred embodiment the cancer is lung cancer.

In another embodiment, the antibody or the ADC of the invention are used in a method for treating and/or preventing cancer. In another embodiment, the antibody or the ADC of the invention are used in a method for treating and/or preventing cancer, wherein the cancer is not related with an increased IL11RA signaling.

The term "cancer" or "tumour" or "tumour disease", as used herein, refers to a broad group of diseases involving unregulated cell growth and which are also referred to as malignant neoplasms. The term is usually applied to a disease characterized by uncontrolled cell division (or by an increase of survival or apoptosis resistance) and by the ability of said cells to invade other neighboring tissues (invasion) and spread to other areas of the body where the cells are not normally located (metastasis) through the lymphatic and blood vessels, circulate through the bloodstream, and then invade normal tissues elsewhere in the body. Depending on whether or not they can spread by invasion and metastasis, tumours are classified as being either benign or malignant: benign tumours are tumours that cannot spread by invasion or metastasis, i.e., they only grow locally; whereas malignant tumours are tumours that are capable of spreading by invasion and metastasis. Biological processes known to be related to cancer include angiogenesis, immune cell infiltration, cell migration and metastasis. Cancers usually share some of the following characteristics: sustaining proliferative signalling, evading growth suppressors, resisting cell death, enabling replicative immortality, inducing angiogenesis, and activating invasion and eventually metastasis. Cancers invade nearby parts of the body and may also spread to more distant parts of the body through the lymphatic system or bloodstream. Cancers are classified by the type of cell that the tumour cells resemble, which is therefore presumed to be the origin of the tumour.

Examples of cancer or tumor include without limitation, breast, heart, lung, small intestine, colon, spleen, kidney, bladder, head, neck, ovarian, prostate, brain, rectum, pancreas, skin, bone, bone marrow, blood, thymus, uterus, testicles, hepatobiliary and liver tumors. In particular, the tumor/cancer can be selected from the group of adenoma, angiosarcoma, astrocytoma, epithelial carcinoma, germinoma, glioblastoma, glioma, hemangioendothelioma, hepatoblastoma, leukaemia, lymphoma, medulloblastoma, melanoma, neuroblastoma, hepatobiliary cancer, osteosarcoma, retinoblastoma, rhabdomyosarcoma, sarcoma, teratoma, acrallentiginous melanoma, actinic keratosis adenocarcinoma, adenoid cystic carcinoma, adenosarcoma, adenosquamous carcinoma, astrocytictumors, bartholin gland carcinoma, basal cell carcinoma, bronchial gland carcinoma, carcinosarcoma, cholangiocarcinoma, cystadenoma, endodermal sinus tumor, endometrial hyperplasia, endometrial stromal sarcoma, endometrioid adenocarcinoma, ependymal sarcoma, Swing's sarcoma, focal nodular hyperplasia, germ cell tumors, glucagonoma, hemangioblastoma, hemangioma, hepatic adenoma, hepatic adenomatosis, hepatocellular carcinoma, insulinoma, intraepithelial neoplasia, interepithelial squamous cell neoplasia, invasive squamous cell carcinoma, large cell carcinoma, leiomyosarcoma, malignant melanoma, malignant mesothelialtumor, medulloepithelioma, mucoepidermoid carcinoma, neuroepithelial adenocarcinoma, nodular melanoma, papillary serous adenocarcinoma, pituitary tumors, plasmacytoma, pseudosarcoma, pulmonary blastoma, renal cell carcinoma, serous carcinoma, small cell carcinoma, soft tissue carcinoma, somatostatin-secreting tumor, squamous carcinoma, squamous cell carcinoma, undifferentiated carcinoma, uveal melanoma, verrucous carcinoma, vipoma, Wilm's tumor.

The term "lung cancer" refers to a heterogeneous group of cancers. The new WHO classification of lung tumours takes into account the great histological heterogeneity in lung cancers. It recognizes several types of lung cancers, such as epidermoid carcinomas, adenocarcinomas, small cell lung carcinomas, large cell carcinomas, large cell neuroendocrine carcinomas, adenosquamous carcinomas, sarcomatoid and pleomorphic carcinomas, non-small cell lung cancer, adenoid cystic carcinoma, mucoepidermoid carcinoma, malignant mixed tumor and several other types.

In a particular embodiment, the lung cancer is selected from non-small cell lung carcinoma (NSCLC), small-cell lung carcinoma (SCLC), adenosquamous carcinoma, carcinoid tumours, bronchial gland carcinomas sarcomatoid carcinoma and lung metastasis.

In the context of the present invention, "metastasis" is understood as the propagation of a cancer from the organ where it started to a different organ. It generally occurs through the blood or lymphatic system. When the cancer cells spread and form a new tumor, the latter is called a secondary or metastatic tumor. The cancer cells forming the secondary tumor are like those of the original tumor. If a lung cancer, for example, spreads (metastasizes) to the bone, the secondary tumor is formed of malignant lung cancer cells. The disease in the bone is metastatic lung cancer and not bone cancer. In a particular embodiment of the method of the invention, the metastasis is lung cancer which has spread (metastasized) to the bone.

### Use in therapy of neutralizing anti-IL-11RA antibodies

In a tenth aspect, the invention relates to a neutralizing antibody which specifically recognizes the interleukin 11 receptor alpha subunit (IL11RA) extracellular domain for use in a method of preventing or treating a disease characterized by increased IL-11RA signaling.

In a particular embodiment, the disease is selected from pulmonary fibrosis, liver fibrosis, liver inflammation, steatosis, steatohepatitis, NASH and cancer. In a more preferred embodiment, the cancer is lung cancer. In yet another embodiment, the cancer is a primary cancer or a metastasized cancer. In yet another embodiment, the cancer is a lung primary cancer or a lung cancer metastasis.

The term antibody has been defined above in the context of the "anti-IL-11RA antibody of the invention" and applies equally to the antibody for use according to this aspect of the invention. In preferred embodiments, the antibody for use according to this aspect of the invention is a monoclonal antibody. In further aspects, the antibody for use according to the present invention encompasses not only full length antibodies (e.g., IgG), but also antigen-binding fragments thereof, for example, Fab, Fab', F(ab')2, Fv fragments, human antibodies, humanized antibodies, chimeric antibodies, antibodies of a non-human origin, recombinant antibodies, and polypeptides derived from immunoglobulins produced by means of genetic engineering techniques, for example, single chain Fv (scFv), diabodies, heavy chain or fragments thereof, light chain or fragment thereof, VH or dimers thereof, VL or dimers thereof, Fv fragments stabilized by means of disulfide bridges (dsFv), molecules with single chain variable region domains (Abs), minibodies, scFv-Fc, VL and VH domains and fusion proteins comprising an antibody, or any other modified configuration of the immunoglobulin molecule that comprises an antigen recognition site of a desired specificity

The "neutralizing antibody against the interleukin 11 receptor alpha subunit (IL11RA) extracellular domain" may be any antibody capable of inhibiting the binding of the receptor and the ligand thereof; for example, an antibody that reacts specifically to the IL-11 receptor, a bispecific antibody that reacts specifically to the IL-11 receptor, an antibody that inhibits an activity of the IL-11 receptor and the like can be mentioned. Thus, the neutralizing antibody is capable of neutralizing the biological signaling activity of IL-11 for example by blocking binding of IL-11 to its corresponding receptor. It will be appreciated that the term 'neutralizing' as used herein refers to a reduction in biological signaling activity which may be partial or complete. In particular a neutralizing antibody according to the invention refers to an antibody which inhibits (partially or completely) the oncogenic properties of IL-11. For example, said neutralizing activities are typically evaluated according to any assay described in the examples.

In a particular embodiment, the disease characterized by an increased IL11RA signaling is cancer. In a preferred embodiment, the cancer is lung cancer, colon cancer, breast cancer, liver cancer, endometrial cancer, stomach cancer and prostate cancer. In a more preferred embodiment, the cancer is lung cancer.

The terms related with this aspect of the invention have already been defined elsewhere and apply equally to the present case.

In another particular embodiment, the neutralizing antibody is the antibody according to the first aspect of the invention.

In a preferred embodiment the neutralizing antibody is used in combination with a chemotherapeutic agent, immunotherapy or targeted therapy.

A "chemotherapeutic agent" is a chemical agent (e.g., compound or drug) useful in the treatment of cancer, regardless of mechanism of action. Classes of chemotherapeutic agents include, but are not limited to: alkylating agents, antimetabolites, spindle poison plant alkaloids, cytotoxic/antitumor antibiotics, topoisomerase inhibitors, antibodies, photosensitizers, and kinase inhibitors.

Targeted cancer therapies are drugs or other substances that block the growth and spread of cancer by interfering with specific molecules ("molecular targets") that are involved in the growth, progression, and spread of cancer. Targeted cancer therapies are sometimes called "molecularly targeted drugs," "molecularly targeted therapies," "precision medicines," or similar names. Many different targeted therapies have been approved for use in cancer treatment. These therapies include hormone therapies, signal transduction inhibitors, gene expression modulators, apoptosis inducers, angiogenesis inhibitors, immunotherapies, and toxin delivery molecules.

Examples of chemotherapeutic agents and targeted cancer therapies have been mentioned within the context to the cytotoxic agents of the ADCs of the invention and are equally applicable to the present case.

Examples of targeted therapy and chemotherapeutic agents include, but are not limited to, Erlotinib (TARCEVA^{®}, Genentech/OSI Pharm.), Bortezomib (VELCADE^{®}, Millenium Pharm.), Fulvestrant (FASLODEX^{®}, Astrazeneca), Sutent (SU11248, Pfizer), Letrozole (FEMARA^{®}, Novartis), Imatinib mesylate (GLEEVEC^{®}, Novartis), PTK787/Z 222584 (Novartis), Oxaliplatin (Eloxatin^{®}, Sanofi), 5-FU (5-fluorouracil), Leucovorin, Rapamycin (Sirolimus, RAPAMUNE^{®}, Wyeth), Lapatinib (GSK572016, GlaxoSmithKline), Lonafarnib (SCH 66336), Sorafenib (BAY43-9006, Bayer Labs.), and Gefitinib (IRESSA^{®}, Astrazeneca), AG1478, AG1571 (SU 5271; Sugen), alkylating agents such as thiotepa and CYTOXAN^{®} cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, triethylenephosphoramide, triethylenethiophosphorarnide and trimethylomelarnine; acetogenins (especially bullatacin and bullatacinone); a camptothecin (including the synthetic analogue topotecan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, -2189 and CB1-TM1); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; antibiotics such as the enediyne antibiotics (e.g calicheamicin, especially calicheamicin gammall and calicheamicin omegall (Angew Chem Intl. Ed. Engl. (1994) 33:183-186); dynemicin, including dynemicin A; bisphosphonates, such as clodronate; an esperamicin; as well as neocarzinostatin chromophore and related chromoprotein enediyne antibiotic chromophores), aclacinoraysins, actinomycin, authramycin, azaserine, bleomycins, cactinomycin, carabicin, canninomycin, carzinophilin, chromomycinis, dactinomycin, daunorubicin, detorubicin, 6-diazo-5-oxo-L-norleucine, ADRIAMYCIN^{®} doxorubicin (including morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptpnigrin, streptozocin, tubercidin, ubenimex, zinostatin, zorubicin; antimetabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti- adrenals such as anunoglutetWmide, mitotane, trilostane; folic acid replenisher such as frolinic acid; aceglatone; aldophosphamide glycoside; aminolevulinic acid; eniluracil; amsacrine; bestrabucil; bisanrrene; edatraxate; defofamine; demecolcine; diaziquone; elfornithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; podophyllinic acid; 2- ethylhydrazide; procarbazine; PSK^{®} polysaccharide complex (JHS Natural Products, Eugene, OR); razoxane; rhizoxin; sizofiran; spirogermanium; tenuazonic acid; triaziquone; 2,2^2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; taxoids, e.g., TAXOL^{®} paclitaxel (Bristol- Myers Squibb Oncology, Princeton, N.J.), ABRAXANE^{™} Cremophor-free, albumin-engineered nanoparticle formulation of paclitaxel (American Pharmaceutical Partners, Schaumberg, Illinois), and TAXOTERE^{®} doxetaxel (Rhone- Poulenc Rorer, Antony, France); chloranbucil; GEMZAR^{®} gemcitabine; 6-thioguanine; mercaptopurine; methotrexate; platinum analogs such as cisplatin and carboplatin; vinblastine; platinum; etoposide (VP-16); ifosfamide; mitoxantrone; vincristine; NAVELBINE^{®} vinorelbine; novantrone; teniposide; edatrexate; daunomycin; aminopterin; xeioda; ibandronate; CPT-11; topoisomerase inhibitor RFS 2000; difluorometlhylornithine (DMFO); retinoids such as retinoic acid; capecitabine; and pharmaceutically acceptable salts, acids or derivatives of any of the above.

Protein kinase inhibitors include tyrosine kinase inhibitors which inhibit to some extent tyrosine kinase activity of a tyrosine kinase such as an ErbB receptor. Examples of tyrosine kinase inhibitors include EGFR- 10 targeted drugs such as: (i) antibodies which bind to EGFR, including MAb 579 (ATCC CRL HB 8506), MAb 455 (ATCC CRL HB8507), MAb 225 (ATCC CRL 8508), MAb 528 (ATCC CRL 8509) (see, US 4943533, Mendelsohn et al.) and variants thereof, such as chimerized 225 (C225 or Cetuximab; ERBITUX^{®}, Imclone) and reshaped human 225 (H225) (WO 96/40210, Imclone Systems Inc.); antibodies that bind type Π mutant EGFR (US 5212290); humanized and chimeric antibodies that bind EGFR (US 5891996); and human antibodies that bind EGFR, such as ABX-EGF (WO 98/50433); (ii) anti-EGFR antibody conjugated with a cyotoxic agent (EP 659439A2); and small molecules that bind to EGFR including ZD1839 or Gefitinib QRESSA^{™}; Astra Zeneca), Erlotinib HC1 (CP-358774, TARCEVA^{™}; Genentech/OSI) and AG1478, AG1571 (SU 5271; Sugen), quinazolines such as PD 153035,4-(3-chloroanilino) quinazoline, pyridopyrimidines, pyrirnidopyrirnidines, pyrrolopyrimidines, such as CGP 59326, CGP 60261 and CGP 62706, and pyrazolopyrimidines, 4- 20 (phenylamino)-7H-pyrrolo[2,3-dJ pyrimidines, curcumin (diferuloyl methane, 4,5-bis (4- fluoroanilino)phthaiimide), tyrphostines containing nitrothiophene moieties; PD-0183805 (Warner-Lambert) and antisense molecules (e.g., those that bind to ErbB-encoding nucleic acid).

In yet another embodiment, additional therapy of the present invention is an anti-tubulin agent. In more specific embodiments, the cytotoxic agent is selected from the group consisting of vinca alkaloids, podophyllotoxins, taxanes, baccatin derivatives, cryptophycin, maytansinoids, combretastatins, and dolastatin. In a more specific embodiment, the cytotoxic agent is vincristine, vinblastine, vindesine, vinorelbine, VP-16, camptothecin, paclitaxel, docetaxel, episilon A, episilon B, nocodazole, colchicine, cortisimide, estramustine, semadotine, discodermolide, maytansine DM-1, auristatin or other dolastatin derivatives, such as auristatin E or auristatin F, AEB, AEVB, AEFP, MMAE (monomethyl auristatin E), MMAF (monomethyl auristatin F), eruterobin or netropsin.

In a preferred embodiment, the additional therapy is selected from Afatinib Dimaleate, Everolimus, Alectinib, Bevacizumab, Brigatinib, Carboplatin, Ceritinib, Crizotinib, Ramucirumab, Dabrafenib Mesylate, Dacomitinib, Docetaxel, Doxorubicin Hydrochloride, Durvalumab, Entrectinib, Erlotinib Hydrochloride, Gefitinib, Gemcitabine Hydrochloride, Lorlatinib, Mechlorethamine Hydrochloride, Trametinib, Methotrexate, Necitumumab, Nivolumab, Osimertinib Mesylate, Paclitaxel, Paclitaxel Albumin-stabilized Nanoparticle Formulation, Pembrolizumab, Pemetrexed Disodium, Selpercatinib, Capmatinib Hydrochloride, Atezolizumab, Vinorelbine Tartrate, Ipilimumab, Etoposide Phosphate, Etoposide, Topotecan Hydrochloride and Lurbinectedin or any combinations thereof.

The term "immunotherapy" has already been described h within the context to the cytotoxic agents of the ADCs of the invention and are is applicable to the present case Thus, the second agent can also be any immunotherapy agent that enhances the anti-cancer effect of the antibodies anti-IL11RA, or an analogue or pharmaceutically acceptable salt thereof. In some embodiments, the second agent is an immunologic agent that can stimulate an effective immune response and/or inhibit immune-suppression. In some embodiments, the second agent is an agent that modulates, particularly inhibits and downregulates immune-suppressive factors such as regulatory T cells and MDSCs, including any inhibitors or antibodies of the programmed cell death 1 ligand 1 (PD-LI)/ programmed cell death protein 1 (PD-i) pathway. In some embodiments, the second agent is any effective inhibitors/antibodies capable of modulating immunocytes activities including but not limited to targeting cytotoxic T lymphocyte-associated antigen 4 (CTLA-4), CD20, CD19, IL-i7a, CD25, arginase I (ARG1), indoleamine-2,3-dioxygenase (IDO), or tryptophan 2,3 dioxygenase (TDO2). In some embodiments, the second agent is BCG. In particular embodiments, the second agent is an antibody against PD-i or an antigen binding fragment thereof. In other particular embodiments, the second agent is an antibody against PD-Li or an antigen binding fragment thereof. In other particular embodiments, the second agent is an antibody against CTLA-4 or an antigen binding fragment thereof. In other particular embodiments, the second agent is a BCG therapy, preferably an attenuated BCG therapy, more preferably mycobacterial cell wall fragments, and most preferably mycobacterial cell wall fragments with biologically active nucleic acids derived from Mycobacterium phlei.

In addition, the present therapy can be administered in conjunction with surgical resection or radiation.

The invention is defined below by the following aspects:
1. A neutralizing antibody which specifically recognizes the interleukin 11 receptor alpha subunit (IL11RA) extracellular domain, wherein said antibody comprises:
   - a heavy chain variable region (VH) wherein the CDR1, CDR2 and CDR3 of the VH comprise, respectively, the sequences of SEQ ID NO: 1, 2 and 3 or functionally equivalent variants thereof, and
   - a light chain variable region (VL) wherein the CDR1, CDR2 and CDR3 of the VL region comprise respectively, the sequences of SEQ ID NO: 4, 5, and 6 or functionally equivalent variants thereof.
2. The antibody according to aspect 1 wherein the FR1, FR2, FR3 and FR4 of the VH region comprise respectively the sequences of SEQ ID NO: 7, 8, 9 and 10 or functionally equivalent variants thereof and/or wherein the FR1, FR2, FR3 and FR4 of the VL region comprise respectively the sequences of SEQ ID NO: 11, 12, 13 and 14 or functionally equivalent variants thereof.
3. The antibody according to any of aspects 1 or 2, wherein the VH region comprises the sequence of SEQ ID NO: 15 or a functionally equivalent variant thereof and the VL region comprises the sequence of SEQ ID NO: 16 or a functionally equivalent variant thereof.
4. The antibody according to aspect 1, which comprises at least one humanized framework region.
5. The antibody according to any of aspects 1 to 4, which is a Fab, a F(ab)2, a single-domain antibody, a single chain variable fragment (scFv), or a nanobody.
6. A nucleic acid encoding an antibody according to any of aspects 1 to 5.
7. An expression vector comprising the nucleic acid of aspect 6.
8. A host cell comprising the nucleic acid of aspect 6 or the expression vector of aspect 7.
9. An antibody-drug conjugate (ADC) comprising:
   (i) a therapeutic agent, and
   (ii) an antibody according to any of aspects 1 to 5.
10. The antibody according to any of aspects 1 to 5 which is coupled to a detectable label.
11. A method for detecting the IL-11 receptor in a sample which comprises contacting the sample with an antibody according to aspect 10 and detecting in said sample the formation of a complex between the antibody and the IL-11 receptor.
12. A pharmaceutical composition comprising the antibody according to any of aspects 1 to 5 or the ADC according to aspect 9 and at least one pharmaceutically acceptable excipient and/or vehicle.
13. The antibody according to any of clams 1 to 5 or the ADC according to aspect 9 for use in medicine.
14. The antibody according to any of aspects 1 to 5 or the ADC according to aspect 9 for use in a method of preventing or treating a disease characterized by increased IL11RA signaling.
15. The antibody or ADC for use according to aspect 14, wherein the disease is selected from pulmonary fibrosis, liver fibrosis, liver inflammation, steatosis, steatohepatitis, NASH and cancer.
16. The antibody or ADC for use according to aspect 15 wherein the disease is cancer.
17. The antibody or ADC for use according to aspect 16, wherein the cancer is lung cancer.
18. A neutralizing antibody which specifically recognizes the interleukin 11 receptor alpha subunit (IL11RA) extracellular domain for use in a method of preventing or treating a disease characterized by increased IL-11RA signaling.
19. The neutralizing antibody for use according to aspect 18, wherein the disease is selected from pulmonary fibrosis, liver fibrosis, liver inflammation, steatosis, steatohepatitis, NASH and cancer.
19. The neutralizing antibody for use according to aspect 18, wherein the disease is cancer.
20. The neutralizing antibody or the antigen-binding fragment thereof for use according to aspect 19, wherein the lung cancer is selected from non-small cell lung carcinoma (NSCLC), small-cell lung carcinoma (SCLC), adenosquamous carcinoma, carcinoid tumours, bronchial gland carcinomas sarcomatoid carcinoma and lung metastasis.
21. The neutralizing antibody for use according to aspects 18 wherein the antibody is as defined in any of aspects 1 to 5.
22. The neutralizing antibody or the antigen-binding fragment thereof for use according to any of aspects 19 to 21, wherein the antibody is used in combination with a chemotherapeutic agent, immunotherapy or a targeted therapy.

The invention will be described by way of the following examples which are to be considered as merely illustrative and not limitative of the scope of the invention.

### EXAMPLES

### METHODOLOGY

### Protein extraction

Protein samples were obtained from cell lines based on a cell culture at 60-80% confluence in a 10-cm or 6-well plate, depending on the experiment. Then, and by working in a cold condition at all times, 2 washes were performed with PBS and the cell content was extracted by adding 200-500 µl of RIPA lysis buffer (Sigma-Aldrich), to which protease and phosphatase inhibitors (Sigma-Aldrich) were added following the concentrations indicated by the manufacturer. The sample was collected by means of cold scraping the plate, and stirring was performed with vortex every 10 minutes for 1 hour. The samples were then centrifuged at 13,000 rpm for 15 minutes at 4°C to remove cell membrane residues. The proteins were kept at -80°C for storage.

In the case of the tumor samples from PDX models, the tumor was extracted, sliced, and crushed using a mortar and liquid nitrogen, and once crushed the RIPA lysis buffer described above was added thereto, and the processing described for the cell lines continued. The added volume of RIPA varied according to the volume of the extracted piece of tumor (500 µl of RIPA for tumors measuring 3-5 mm3).

For protein extraction from organoid samples, culture medium was removed from the plate and 2 ml of cold Cell Recovery solution (Corning) were added. After the dissolution of the matrigel (BD), cells were separated by pipetting and collected in a tube, where they were cold incubated for 30 minutes. The cell pellet was collected after centrifugation at 4°C, at 1500 rpm, for 5 minutes and 2 washes were performed with 1 ml of cold PBS. The cell pellet was resuspended in 200 µl of RIPA and processed in the same manner as samples obtained from cell lines and PDX.

### Protein quantification

To measure the total amount of protein of the extracts, 200 µl of Bradford reagent 1X (BioRad) were added in a 96-well plate mixed with 2 µl of samples and absorbance at 595 nm was measured with the Victor Multilabel Plate Reader (PerkinElmer). The protein concentration of a standard curve was measured in parallel using known amounts of serum albumin (BSA) to enable extrapolating the concentrations of the samples of interest.

### Western blot

### - Sample preparation:

After determining the total amount of proteins of the samples, aliquots containing 50-100 µg of total protein were prepared according to the protein to be analyzed and Laemmli loading buffer 5X was added (Tris-HCl 62.5 mM pH 6.8, 10% glycerol, 1% SDS, 5% 2-mercaptoethanol, 0.0025% bromophenol blue (Sigma-Aldrich)). The samples were denatured by incubating them at 95°C for 5 minutes.

### - Polyacrylamide gel electrophoresis:

Acrylamide electrophoresis gels were resolved using the Mini-Protean Tetra Cell system (BioRad). Precision Plus Protein Dual Color (BioRad) was used as protein molecular weight standard.

The separating gel was prepared in buffer (100 mM Tris-HCI pH 8.8, 0.1% SDS) with 7.5, 10, or 12.5% of polyacrylamide based on a 40% acrylamide:bisacrylamide ratio of 37.5:1 (BioRad), according to the size of the proteins to be analyzed. The sample-concentrating gel was prepared with 5.25% of polyacrylamide in buffer (125 mM Tris-HCI pH 6.8, 0.1% SDS). The gels were polymerized with 0.1% APS using TEMED as catalyst. Electrophoresis was carried out at 100 V for about 2 hours depending on the percentage of polyacrylamide of the separating gel. Tris/Glycine/SDS (BioRad) was used as electrophoresis buffer for electrophoresis development.

### - Transfer:

After separating the proteins by molecular weight in the polyacrylamide gel, said proteins were transferred to a PVDF (polyvinylidene fluoride) membrane (GE Healthcare) previously wetted with methanol, using the Mini Trans-Blot Cell system (BioRad) and the transfer buffer Tris/Glycine (BioRad) with 20% methanol (Merck-Millipore).

The gel, together with the membrane, were inserted in a cassette between two pieces of Whatman paper soaked in transfer buffer, and the latter was included in the transfer cuvette. The method was carried out in cold under constant amperage conditions (300 mA) for 3 hours.

### - Membrane blocking and antibody incubation:

The membrane containing the transferred proteins was blocked for 2 hours with a TBS solution with 0.1% Tween20 (Sigma-Aldrich) (TBST) containing 2-5% BSA, depending on the antibody to be used (Labotac). The membrane was then incubated with the primary antibody, diluted in blocking buffer, O/N, at 4°C. The next day, after three 5-minute washes with TBST, it was incubated for 1 hour with the corresponding secondary antibody diluted in blocking buffer. Finally, the membrane was again washed 3 times in TBST buffer and developed with the Clarity ECL reagent (BioRad) in a ChemiDoc XRS+ equipment (BioRad).

Generally, the membranes were cut for incubation with different antibodies and for viewing proteins of different sizes. When the sizes of the proteins were similar and there was not enough sample to load several gels, the stripping technique was used. To that end, the membrane was incubated for 45 minutes with a solution containing 10% SDS, 0.5M TrisHCI pH6.8, and 4% 2-mercaptoethanol, and then washed every 10 minutes with TBST for 30 minutes. The membrane was then blocked again, and the new protein of interest was viewed in the manner as described above.

### - Band quantification:

ImageLab 5.0 (BioRad) software was used to quantify the protein bands of the membranes. The band volume of the protein of interest was quantified and normalized with the band volume corresponding to the constitutively expressed protein (β-actin or α-tubulin, depending on the assay).

### Inmunohistochemistry (IHC)

The immunohistochemistry of xenograft tumors from PDX was performed by the histopathology unit of the National Cancer Research Center (Centro Nacional de Investigaciones Oncologicas - CNIO). The samples were fixed in 10% formalin, paraffin-embedded, and sectioned to 3 µm. For immunohistochemical staining, an automatic platform (Ventana Discovery XT, Roche or Autostainer Link, Dako) was used, using the reagents of the manufacturer. After paraffin removal and antigen retrieval with the selection buffer, the primary antibody was incubated using a system based on a detection multimer, anti-rabbit OmniMap-HRP or Novolink polymer, and an anti-mouse bridge if the primary antibodies were obtained from a mouse. ChromoMap DAB included in the kit for the peroxidase-bound multimer was used for chromogenic development. Finally, counterstaining was performed with hematoxylin for 5 minutes and the sample was dehydrated for mounting.

### Generation of anti-IL-11RA neutralizing antibody

IL-11RA neutralizing antibody was generated in collaboration with the Crystallography and Protein Engineering Unit and the Monoclonal Antibody Unit of the CNIO. The antigen required for inducing immune response in the animal was the extracellular domain of the human IL-11RA protein fused to an Fc moiety and containing an hexahistidine tag.. This construct was produced by the Crystallography and Protein Engineering Unit of the CNIO in HEK293 mammalian cells and purified by means of protein-A affinity chromatography, followed by gel filtration, as indicated below.

### - Generation of expression vectors

The sequence encoding the extracellular domain (ECD) of human IL11RA (NP_001136256, residues 1-365) was fused to a C-terminal human IgG1 Fc sequence in a CMV-based pcDNA3 vector by PCR amplification using pCMV6-IL11RA (IL11RA (Myc-DDK-tagged)-Human interleukin 11 receptor, alpha (IL11RA), transcript variant 3; NM_001142784) from Origene CAT#: RC226571 as template DNA and the oligos IF-KpnI-hIL11RA-5' (5'-GACCCAAGCTTGGTACCACCATGAGCAGCAGCTGCTCAGGGCTGAGCAG-3') and IF-IL11RA365-IEGRMD-BamHI-3' 
(GTCACAAGATTTGGATCCTGGATCTCGGCCTTCGATATCCTGCTCCACAGAGTCC
CTGTGATCAAGTAGCCGAGGGTGTGGTTG).
 PCR product was cloned into pcDNA3-Fc vector, digested with Kpnl and BamHI, by means of In-Fusion^{®} reaction (Takara). The resulting expression vector contained the endogenous IL11RA signal peptide, the IL11RA ECD, an IEGRMD linker and the Fc region. Following verification of DNA sequence, pDNA3-IL11RA-Fc expression plasmid was purified for transient transfection experiments using PureLink^{™} HiPure Plasmid Filter Maxi-Prep kit (Invitrogen).

### - Expression and purification of IL11RA-Fc

Expi293 cells (ThermoFisher) were used to express the recombinant protein. Those cells were grown in Expi293 medium (ThermoFisher) at 37°C, 120 rpm, 8% CO2 atmosphere with 80% humidity in plastic flasks with ventilated caps (Corning^{®} Erlenmeyer sterile polycarbonate with 0.2 µm ventilated caps). For expression, pDNA3-IL11RA-Fc (1mg/L of final culture volume) was diluted in Opti-MEM medium (Gibco) and mixed with ExpiFectamine 293 Reagent (ThermoFisher) according to the manufacturer's protocol. After 20-min incubation at room temperature, the transfection complexes were added to the cells and ExpiFectamine 293 Transfection Enhancers 1 and 2 were added 20 h post-transfection. The cell culture was cultured in a shaking incubator (120 rpm) at 37°C for 6 days. Cells were then pelleted by centrifugation at 10000g for 15 min and the supernatant was loaded onto a 5-ml HiTrap Protein A HP column (GE Healthcare) in an ÄKTA prime chromatography system (GE Healthcare), equilibrated with PBS, pH 7,4. Bound protein was eluted with 0.1 M Glycine-HCI pH 3.0 and elution fractions were immediately neutralized with 1 M Tris-HCI pH 8.8. Protein-containing elution fractions were pooled, exchange into PBS, pH 7,4 using a HiPrep 26/10 Desalting Column (GE Healthcare) in an ÄKTA prime (GE Healthcare) and concentrated using a Vivaspin 15R centrifugal filter (Sartorius) with a molecular weight cut-off of 30 kDa. Aliquots of the purified protein were flash-frozen in liquid nitrogen and stored at -80°C. Recombinant proteins were analysed by SDS-PAGE using Mini-Protean^{®}TGX^{™} 4-20% gradient gels (Bio-Rad) and IL11RA was identified by Western-blot with an anti-IL11RA monoclonal antibody and by tryptic digestion for mass spectrometry analysis.

### - Immunization and fusion process

Wistar strain rats were then immunized with the antigen that was produced by means of 3 intraperitoneal injections of 100 µg of protein.

The animal was sacrificed after 3 days and the spleen, which was found to house a large population of antibody-producing B lymphocytes, was extracted. Rat splenocytes were separated and fused with the myeloma cell line of NS-1 mouse by means of using polyethylene glycol, giving rise to hybridomas capable of producing antibodies and proliferating indefinitely. The colonies formed by the hybridomas were tested after 10 days to confirm the production of the antibody of interest by means of ELISA and IHC. The colonies producing the antibody of interest were cloned to ensure that the obtained antibody was produced from a single hybridoma, giving rise to a monoclonal antibody.

IL-11 RA recognition of the obtained antibody was validated by means of IL11RA-Fc-HIS protein-covered ELISA plates, as well as with cytospins of HEK293T cells transfected with pCMV6-IL11RA plasmid (Origene), and by means of WB in cells with and without the over-expression of IL-11RA (antibody diluted at 1:500).

The supernatant obtained from the selected clone was purified by means of affinity chromatography using a protein G column coupled to AKTA prime equipment. The obtained fractions were dialyzed overnight against PBS and quantified by means of NanoDrop^{™} 2000/2000c (Thermo Fisher Scientific).

### Generation of organoids from PDX models (3D cell cultures)

To generate organoids derived from PDX (Patient Derived Xenograft) tumors, tumor was first extracted from the mouse and sliced into fragments measuring 1-2 mm3; these fragments were transferred to a tube with 5 ml digestion solution made up of 1.5 mg/ml collagenase IA (Sigma-Aldrich) in DMEM/F12 culture medium (Sigma-Aldrich) supplemented with 1 Mm HEPES (Gibco), GlutaMax 1X (Sigma-Aldrich), and 0.25% primocin (Invivogen). The sample was incubated for 1 hour at 37°C and passed through a 70 µm filter. Blood cells were lysed using EasySep lysis buffer (Stemcell technologies). The cell pellet was resuspended in matrigel (BD) containing 120,000 cells in 300 µl of matrigel and transferred dropwise to 6-well plates temperature-adjusted to 37°C. When the matrigel solidified, DMEM/F12 medium supplemented with 1 mM HEPES, GlutaMax 1X, 2% FBS, 0.25% primocin, 5 µg/ml insulin (Sigma-Aldrich), 3 ng/ml EGF (Life Technologies), 1 µg/ml hydrocortisone (Sigma-Aldrich), B27 1X (Life Technologies), and 10.5 µM Rho kinase inhibitor (Life Technologies), was added. The organoids were incubated at 37°C, changing the medium every 4 days.

### Verification of the neutralizing action of anti-IL-11RA antibody in-vitro

To verify the neutralizing action of this antibody in-vitro, 250,000 cells from the A549 cell line were seeded in 6-well plates, and after 24 hours, the cells were kept for 5 hours without FBS. After that period, 3 of the wells were incubated for 24 hours at 37°C with increasing concentrations of neutralizing antibody (0, 2, and 40 µg/ml). The next day, 50 ng/ml of IL-11 were added. Protein extracts were obtained 15 minutes after stimulation with rhIL-11 for the subsequent verification of STAT1 and STAT3 activation level by means of WB. For the antibody to be considered a valid neutralizing antibody, a decrease in STAT protein phosphorylation must occur upon stimulation with rhIL-11 cells incubated with the antibody. Concentration of 40 µg/ml was selected for treating organoids obtained from PDX, and for thereby verifying the neutralizing effect of the antibody in In-vitro 3D models.

### Animal testing techniques

All the procedures performed with animals were carried out in the animal laboratory of the CNIO and were approved by the Animal Protection Committee of the Autonomous Community of Madrid (PROEX 084/15 and PROEX069/19).

### - Patient-derived tumor xenografts (PDSs)

Hsd:Athymic Nude-Foxn1<nu> immunodeficient or athymic nude mice (Charles River Laboratories) were also used to obtain PDX (Patient Derived Xenograft) models.

### - Establishing PDX model collection

A collection of 13 PDX models of lung adenocarcinoma was used. To generate lung adenocarcinoma, tumors from patients operated in Hospital Universitario Virgen del Rocío of Seville, implanted in immunodeficient mice in the animal laboratory of Institute of Biomedicine of Seville (IBIS) (CEI: 2013 PI1277; SSA/SI IMDIpdm) were used. This collection was then transferred to the CNIO where it is now kept and used for different projects (CEI 16/355; PROEX 084/15).

Small fresh fragments of these tumors, which were embedded in matrigel (BD) and implanted in the flank of 5-8 week old immunodeficient mice anesthetized by means of isoflurane inhalation and analgesized with buprenorphine, were used to establish the PDXs. To perform the implantation, a small cut was made in the flanks of the mouse and a tumor fragment having a volume of 50-100 mm3 was inserted. The follow-up of the size of the tumors was performed every week with a digital caliper. Once the tumors reached a volume of 1000 mm3, the animal was sacrificed and tumors were extracted, sectioned into different fragments for implantation in more mice and for model expansion, and for later cryopreservation and implantation, thereby being able to perform different treatments. Furthermore, some fragments were fixed with formalin and embedded in paraffin (blocks obtained by the Histopathology Unit of the IBIS and CNIO), or frozen for subsequent DNA, RNA, and protein extraction.

The PDX models were characterized to determine the expression of the proteins of interests.

### - Treatment of PDX models with the IL-11RA neutralizing antibody

After validating the neutralizing activity of the anti-IL-11RA antibody in-vitro, said anti-IL-11RA antibody was used for studying the effect thereof on PDX models. The selected model was implanted in 18 mice which were then distributed into 3 groups of 5 mice: a control group, which was treated with a vehicle (physiological saline), a group treated with anti-IL-11RA (300 µg/mouse), and a group treated with the control anti-GST (glutathione-S-transferase) antibody (300 µg/mouse). Intraperitoneal injections were performed 3 times a week for 3 consecutive weeks. At the end of treatment, the mice were sacrificed, and the tumors processed and stored for subsequent analysis.

Graphs of the relative growth of the tumors over the course of treatment were represented, relativizing the growth of the tumors with their growth on the first day of treatment.

### Statistical analysis

Statistical analyses were performed using SPSS (IBM) or GraphPad Prism statistic software packages, using the statistical tests described in detail below to obtain the p-values. Values below 0.05 (* (p<0.05), ** (p<0.01), ***(p<0.001)) were considered statistically significant.

### - Analysis of in vitro and in vivo experiments

To analyze whether or not the differences between the different conditions tested in the in-vitro experiments were statistically significant, non-parametric Mann-Whitney U or Kruskal-Wallis tests were performed (depending on the number of conditions to be compared) in the values corresponding to the independent biological replicates of the experiments. To analyze whether or not the differences in tumor growth of the different experimental conditions in the in-vivo assays were statistically significant, the same tests were performed in the values corresponding to tumor size normalizations.

### RESULTS

### Example 1: Characterization of the antibody

After the fusion, the LAU490A antibody was selected and purify for in vivo experiments due to its neutralizing capacity (Figure 2)

As shown in figure 1, the selected antibody, LAU490A, is able to recognize IL11RA protein in HEK-IL11RA-MYC transfected cells by immunocytochemistry, ELISA technique and western blotting (Figure 1). The anti-IL-11RA antibody was validated through verification of the detection of IL-11RA in the cell line that overexpressed same (Figure 2A).

### Example 2: verification of its neutralizing action

The neutralizing effect of the antibody in the JAK/STAT pathway *in-vitro* was verified both in cell lines in 2D growth (Figure 2B) and in PDX derived organoids (3D) (Figure 2C). To that end, the cells and organoids were treated for 24 hours with the antibody for their subsequent stimulation with rhIL-11 for 15 minutes and for subsequent STAT1 and STAT3 protein activation analysis (Figure 2B-C). In the cell lines and organoids treated with the antibody, STAT1 and STAT3 phosphorylation caused by IL-11 was inhibited, so the blocking effect thereof on the in-vitro pathway was confirmed.

### Example 3: Role of IL-11 as therapeutic target

After verifying that the antibody had a neutralizing effect in-vitro, PDX57 model which expressed IL-11 and its receptor but did not exhibit a high baseline activation of the JAK/STAT pathway, was chosen to perform the in-vivo treatment (Figure 3). The model used maintained the histology of the adenocarcinoma of origin. Treatments on the selected model were performed based on a mouse with grown tumors which were extracted for the subcutaneous implantation of fragments in both flanks of 18 athymic mice. When these tumors reached a volume of 200 mm³, the animals were distributed into 3 groups of 5 animals: a control group, to which only vehicle was administered, a group treated with anti-IL-11RA (300 µg/mouse), and a third group treated with anti-GST (glutathione-S-transferase) antibody (300 µg/mouse) as specificity control. Treatment was performed intraperitoneally, and 3 doses were administered every week for 3 weeks (Figure 4B). The tumors were measured every 5 days with a precision caliper and the size of each tumor was normalized with the size it had on the day the treatment started. As a result of the treatment, it was observed that the IL-11RA neutralizing antibody significantly reduced the size of the tumors (Figure 3C). IHC staining showed that treatment with the anti-IL-11RA neutralizing antibody reduced STAT3 protein activation and the expression of cyclin D1 and Ki67 proliferation proteins (Figure 3D).

This result confirms that the inhibition of the IL-11 pathway could be a suitable therapeutic strategy for lung adenocarcinoma tumors with high levels of this protein.

## Claims

1. A neutralizing antibody which specifically recognizes the interleukin 11 receptor alpha subunit (IL11RA) extracellular domain, wherein said antibody comprises:
- a heavy chain variable region (VH) wherein the CDR1, CDR2 and CDR3 of the VH comprise, respectively, the sequences of SEQ ID NO: 1, 2 and 3 or functionally equivalent variants thereof, and
- a light chain variable region (VL) wherein the CDR1, CDR2 and CDR3 of the VL region comprise respectively, the sequences of SEQ ID NO: 4, 5, and 6 or functionally equivalent variants thereof.

2. The antibody according to claim 1 wherein the FR1, FR2, FR3 and FR4 of the VH region comprise respectively the sequences of SEQ ID NO: 7, 8, 9 and 10 or functionally equivalent variants thereof and/or wherein the FR1, FR2, FR3 and FR4 of the VL region comprise respectively the sequences of SEQ ID NO: 11, 12, 13 and 14 or functionally equivalent variants thereof.

3. The antibody according to any of claims 1 or 2, wherein the VH region comprises the sequence of SEQ ID NO: 15 or a functionally equivalent variant thereof and the VL region comprises the sequence of SEQ ID NO: 16 or a functionally equivalent variant thereof.

4. The antibody according to any of claims 1 to 3, which comprises at least one humanized framework region.

5. A nucleic acid encoding an antibody according to any of claims 1 to 4, an expression vector comprising said nucleic acid or a host cell comprising said nucleic acid or said expression vector of aspect.

6. An antibody-drug conjugate (ADC) comprising:
(i) a therapeutic agent, and
(ii) an antibody according to any of claims 1 to 4.

7. The antibody according to any of clams 1 to 4 or the ADC according to claim 6 for use in medicine.

8. The antibody according to any of claims 1 to 4 or the ADC according to claim 7 for use in a method of preventing or treating a disease **characterized by** increased IL11RA signaling.

9. The antibody or ADC for use according to claim 8, wherein the disease is selected from pulmonary fibrosis, liver fibrosis, liver inflammation, steatosis, steatohepatitis, NASH and cancer.

10. The antibody or ADC for use according to claim 9 wherein the disease is cancer.

11. The antibody or ADC for use according to claim 10, wherein the cancer is lung cancer.

12. A neutralizing antibody which specifically recognizes the interleukin 11 receptor alpha subunit (IL11RA) extracellular domain for use in a method of preventing or treating a disease **characterized by** increased IL-11RA signaling.

13. The neutralizing antibody for use according to claim 12, wherein the disease is selected from pulmonary fibrosis, liver fibrosis, liver inflammation, steatosis, steatohepatitis, NASH and cancer.

14. The neutralizing antibody or the antigen-binding fragment thereof for use according to claim 13, wherein the cancer is a lung cancer is selected from non-small cell lung carcinoma (NSCLC), small-cell lung carcinoma (SCLC), adenosquamous carcinoma, carcinoid tumours, bronchial gland carcinomas sarcomatoid carcinoma and lung metastasis.

15. The neutralizing antibody for use according to any of claims 12 to 14 wherein the antibody is as defined in any of claims 1 to 4.
